# EUROPEAN PATENT APPLICATION

(11) **EP 2 106 803 A1**
(43) Date of publication of application: **07.10.2009**
(21) Application number: 08103387.0
(22) Date of filing: 04.04.2008
(51) Int. Cl.: A61K 39/21, G01N 33/50

(54) **Method to design and uses of overlapping peptides for monitoring T-cell responses in HIV patients**

(71) Applicant: Fondazione Centro San Raffaele del Monte Tabor, 20132 Milano (IT); Helmholtz Zentrum München Deutsches Forschungszentrum für Gesundheit und Umwelt (GmbH), 85764 Neuherberg (DE)
(72) Inventor: Malnati, Mauro Severo, 20132 Milano (IT); Poli, Guido, 20132 Milano (IT); Lusso, Paolo, 20132 Milano (IT); Cosma, Antonio, 81927 Munich (DE); Erfle, Volker, 81679 Munich (DE)
(74) Representative: Capasso, Olga

(57) **Abstract**

The present invention relates to a method for designing a set of overlapping immunogenic peptides of a variable antigen, set of overlapping immunogenic peptides obtained and uses thereof, in particular for diagnostic and therapeutic purposes. The present invention relates also to a sub-population of CD8 T cells associated with the non-progressive status in HIV-infected subjects.

## Description

### FIELD OF THE INVENTION

The present invention relates to a method for designing a set of overlapping immunogenic peptides of a variable antigen, the set of overlapping immunogenic peptides obtained and uses thereof, in particular for diagnostic and therapeutic purposes. The present invention relates also to a sub-population of CD8 T cells and its use for the diagnostic of HIV infected subjects.

### BACKGROUND OF THE INVENTION

The growing knowledge of how the human immune system deals with different types of pathogens is allowing to better define the immune correlates that leads to the protective effects exerted by efficacious vaccines. Therefore, delineation of the immune correlates of protection occurring during natural infection and after vaccination has become a fundamental step in the development of new vaccines. Important steps toward the identification of the immune correlates relevant for protection from HIV have been made mimicking HIV-1 infection in animal models and studying the natural course of the HIV-mediated disease in different cohorts of HIV-seropositive individuals. Indeed, studies of passive immunization and immune depletion in animal models strongly suggest that both humoral (neutralizing antibodies) and cell-mediated immune responses may provide effective protection from infection and disease progression in HIV-1 infection (3, 24).

Indeed, induction of strong and durable T lymphocytes responses against HIV-1-infected cells has become an important immunogenicity outcome in vaccine trials, given the increasing evidence that HIV-1-specific cell-mediated immune responses partially protect macaques experimentally infected with SHIV (4, 24) and contribute substantially to control HIV-1 disease in humans (16, 18, 20, 22). Therefore, increasing efforts are directed towards the development of effective vaccines through induction of virus-specific T cell responses, which play a significant role in determining viral set point in HIV infection.

In recent years, methods for detecting both CD4 and CD8 T cell responses have been significantly improved by the introduction of cytokine enzyme-linked immonospot assays (particularly IFN-γ Elispot) and intracellular cytokine staining (ICS) assays (6, 23). This has led to more sensitive detection and quantification of the cellular immune response but, despite greater ease and rapidity of assessment these approaches have left a number of issues unresolved and there is not yet a consensus on the best methods for assaying the magnitude and the breadth of these responses. In this regard, two mayor issues are still debated among scientists: the choice of the HIV-1 sequence/s to use for the generation of the screening peptides since HIV is a highly variable virus, and the best type of approach for defining cytotoxic T lymphocytes (CTL) and T-helper epitopes. Indeed, differences in sequence between two HIV-1 isolates within the same clade range between 7-15% and between viruses belonging to different clades can be as high as 30%. Constructing a consensus sequence may represent a compromise for this problem (13), although it has to be kept in mind that it is very difficult to generate artificial sequences at the hyper-variable regions and that viral escape from immune detection has been consistently linked to amino acid substitutions in HIV-1 T cell epitopes (10, 19). Regarding the second issue, two are the strategies currently used to define T cell epitopes: bioinformatics approach which utilizes predictive algorithms containing peptide-binding 'motifs' and 'supermotifs' for the major histo-compatibility complex (MHC) class I and II molecules to predict epitope (9) or via overlapping peptides that span the viral proteins of interest (2, 6, 10). In this latter case the length and degree of overlap of peptides are further motif of scientific debate (6, 10, 11). The use of computer-assisted bioinformatics approaches, based on HLA peptide binding motifs and available viral sequences, has been shown to be a useful tool for identifying CTL epitopes derived from HIV-1(9). However, since binding motifs have not yet been defined for all HLA alleles, HLA profiling of population belonging to developing countries is scanty and peptide binding is not the sole determinant of epitope dominance, not all CTL epitopes will be identified by HLA motif-screening algorithms. On the other hand, the methods based on a comprehensive screening of HIV-1 encoded antigens via overlapping peptide pools are attractive because makes no assumptions about peptides that do not conform exactly to the peptide-binding motif, although they still do not detect all the T cell responses present in HIV-infected individuals (5). The main limitation to this type of screening assays is that a consensus on the best design with regards to peptide length and overlap has not been reached yet. Indeed, the length and degree of peptide overlap not only influence the detection of T cell responses but also has a major impact on cost, labor intensiveness and amount of blood needed to perform such studies.

WO0116163 discloses a peptide mixture, a pharmaceutical composition and a vaccine against a chronic infection caused by a virus comprising a mixture of 10 to 30 amino acids (aa) long peptides each with a 5 to 25 aa overlap of the adjacent overlapping peptide spanning the amino acid sequence of a viral protein of said virus, e.g. Hepatitis B, Hepatitis C, GBvirus-C, HIV and Herpes viruses. Hepatitis B has been used as an example and it is demonstrated that a peptide mixture composed of seventeen 20 to 23 aa long peptides spanning the amino acids 1 to 183 of the hepatitis B core antigen (HBcAg) could activate specific T cells regardless of the host MHC/HLA genotype that recognize the native protein processed by professional antigen presenting cells (APCs). Further, a method is described for the treatment of a viral infection, particularly a non-resolving chronic viral infection, making use of the novel peptide mixture immunogen.

WO2004031345 provides compositions containing HIV epitopes, which are recognized by cytotoxic T lymphocytes (CTL). Such polypeptides are used in vaccines and immunotherapies. HIV-1 epitopes represent early targets in a naturally-occurring response against HIV-1 infection. In particular, an immunogenic composition comprising an HLA class I restricted HIV-1 polypeptide, wherein said polypeptide consists essentially of 8-12 amino acids and wherein said peptide is characterized as an early CTL target in HIV-1 infection is claimed.

WO2005015207 discloses a method and diagnostic tests based on flow cytometric analysis of antigen-specific T lymphocytes. However the document does not define a specific population of T-cells and the method does not allow the diagnostic of the non progressive status in an HIV-1 infected subject.

Vardas et al. (26) discloses a method for designing HIV Tat specific overlapping peptides for HIV vaccine trials.

There is the need for identifying an optimal peptide design strategy. Optimal epitopes are protein fragments that are best recognized by T lymphocytes due to their efficient binding. Optimal CTL epitopes are 8-11 amino acid residues in length, and, therefore, an overlap of at least 10 residues between adjacent overlapping peptides is required to ensure that no 11-mer is missed in the screening process. Increasing the number of overlapped amino acids (11 vs 10 amino acids) has not produced a clear advantage in detecting CTL responses (10), beside a substantial increase in the number of peptide necessary to perform a complete HIV-1 peptide-scan (746 vs 600). This leads to a 20% increase in the cost of the assay and on the amount of cells needed, thus reducing the feasibility of such approach. A documented disadvantage of overlapping peptides is that epitopes situated in the middle of longer peptides may not be always detectable because of suboptimal epitope presentation. This implies that longer peptide sets (20 mers) may be less sensitive in detecting CD8 T cell responses (6, 10), whereas they seems to be more efficient in resolving Class II restricted CD4 responses (10). Indeed, a CD8 T-cell response to an epitope present within a longer peptide is best detected when the epitope is situated at the peptide C-terminus, both when peptides were designed to include the optimal epitope at every possible position and when responses towards optimal epitopes and corresponding overlapping peptides were compared in a larger group of subjects (10). Furthermore, even overlapping 15-mers are less efficient than optimized epitopes in detecting low frequency CD8 responses (200 SFU/million PBMC), when the epitope is located centrally in the peptide (5).

Taking advantage of the well establish notion that only a minority of the 20 possible amino acidic residues at the peptide C-terminus, a primary anchor position that contributes substantially to binding of the peptide to the MHC class I molecule will be present in CTL epitopes (only 9 of them served as the C-terminal anchor position in 96% of described optimal epitopes and in 95% of peptide-binding motifs described for over 60 HLA class I alleles), a novel strategy that combines database information with the systematic experimental procedure has been recently experimented (10). The direct comparison between gold standard 15/11 and 15/10 overlapping residue peptide sets has demonstrated the efficacy of this approach that, allowing the use of longer peptide (18mers) without losing in detection of magnitude and breath of the immune response, permits a conspicuous reduction in the number of peptide needed (410 vs 746 for a complete HIV-1 genome scanning). Although an optimal overlapping set has still to be obtained (13% of the CTL responses were not shared between different peptide sets), this result suggests that more rational approaches to overlapping peptides design are feasible though, in some instances, the number of peptide needed is greatly increased (17).

### SUMMARY OF INVENTION

In the present invention, the authors present a new strategy of T-cell epitope screening that, without compromising the detection of the full-range of CTL and T-helper responses, limits both the amount of human material and assay cost.

Departing from the teaching of Vardas et al. (26) the authors optimized a method of peptide designing and were able to set up different set of peptides. These peptides were able to identify a new T-cell population exclusively associated with the control of the HIV-infection in long term non progressors (LTNP).

The method was used to generate two sets of overlapping peptides derived by the HIV-1 regulatory antigens Tat and Nef that are superior in detecting CD8 CTL responses without loosing the ability to uncover the CD4 T-helper responses. Moreover, the new peptides pools were used together with an immunocytofluorimetric assay to investigate the T-cell immune response of a selected group of HIV-infected individuals. This approach allowed the identification of a new T-cell population exclusively associated with the control of the HIV-infection in long term non progressors (LTNP).

Therefore, the authors propose the new peptide strategy for designing a feasible strategy for the screening of a significant number of HIV candidate vaccine antigens in large cohorts of European and African individuals, and the use of the new peptide pools combined with the immunocytofluorimetric technique for the identification of the peculiar T-cell population that act as a marker to monitor efficacious anti-HIV T-cell responses.

According to one aspect, the invention consists in a new method (called Variable Overlapping Peptide Scanning Design, VOPSD) to design overlapping peptides (10 to 19 aa long). Such peptides may be used for the detection of both CD4 and CD8 T-cell responses derived from any type of well defined microbial or tumor derived antigen. Given the superior ability of the VOPSD strategy to detect T-cell responses in humans the peptides sets generated following this method possess several relevant industrial applications. As a matter of facts, they can be used as a complete set, covering the complete sequence of a known antigen, for screening system to individuate the human T-cell mediated immune responses relevant for the development of T-cell based vaccines. The peptides may be used as vaccine components and they can be the major components of diagnostic kits for the detection of T-cell based immune responses. In particular the sets of peptides generated using the HIV-1 encoded Tat and Nef antigens belonging either to clade B or C can be used as above described. The present invention discloses also a novel CD8 T-cell population exclusively present in HIV-infected subjects with a non-progressive course of the HIV disease. The detection of such a population will allow the monitoring of HIV-infected individuals for the presence of an efficacious anti HIV-1 T-cell response. A kit able to detect this population will be useful to evaluate, in HIV-seronegative individuals subjected to preventative anti HIV-1 vaccination programs, the elicitation of anti HIV-specific protective CD8 T-cell responses and, in HIV-infected individuals subjected to therapeutic vaccination protocols with HIV-encoded antigens, the appearance/increase of anti HIV-specific protective CD8 T-cell responses.

In addition, a kit able to measure such population will be useful to evaluate in patients naïve for antiretroviral therapy (ART) the timing for the therapeutic intervention.

Therefore it is an object of the present invention a method for designing a set of overlapping peptides of an antigen comprising the steps of:
a) aligning the amino acid sequence of a variant of the antigen with one amino acid consensus sequence of the same antigen;
b) mapping on said aligned amino acid sequences known immunodominant regions and optimal epitope regions;
c) individuating a first sub-set of peptides consisting of conserved sequence regions between the amino acid sequence of the variant and the amino acid consensus sequence of the variable antigen, said peptides being longer than 10 amino acids ;
d) completing the overlapping peptide design by individuating a second sub-set of peptides, each peptide containing a protein fragment of the amino acid sequence of the variant that differs for one or more amino acid residues from the amino acid consensus sequence of the variable antigen, and optionally one or more immunodominant region and/or a optimal epitope region or portions thereof at its N or C terminus,
wherein each of said peptides of said second sub-set of peptides is between 10 and 19 amino acids long and does not have as the last C-terminal amino acid any of the following amino acids: Asn, Asp, Gln, Glu, Gly, His, Ser;
and wherein each of the set of overlapping peptides has a gap not longer than 8 amino acids with its closest overlapping peptide.

The wording "gap" refers to the fact that the following peptide starts between 0-8 aa. downstream of the aa. protein sequence. It is a common terminology for people experienced in the field.

With respect to the method disclosed by Vardas et al. (26), the method comprises new features providing unexpected properties of the designed peptides. The length of peptides was optimized (between 10 and 19 amino acids) and it was found that each of the set of overlapping peptides should have a gap not longer than 8 amino acids with its closest overlapping peptide. Moreover, overlapping peptides must contain at their termini previously characterized T-cell epitopes or immunodominant fragments. The choice for positioning such epitopes in the N- terminus or in the C-terminus of the longer scanning peptides is based upon the evaluation of the HLA binding motifs proper of the epitope under investigation. Furthermore, it is also new with respect to Vardas et al (26) that, when different optimal epitopes are partially overlapped in the protein sequence, the choice for the best placement in the C-terminus of the epitopes is dictated by the frequency in the human population of the HLA allele/s restricting the epitope recognition and by the possibility to place it, as an alternative, to the N-terminus of a different scanning peptide.

Preferably, the variable antigen is an HIV antigen. More preferably the HIV antigen is an HIV-1 antigen. Still preferably the HIV antigen is an HIV-1 Tat antigen or an HIV-1 Nef antigen.

It is an object of the invention a set of overlapping peptides obtainable according to the method of the invention. Preferably the set of overlapping peptides is of the variable Tat antigen of HIV-1. More preferably, the set of overlapping peptides consists of peptides belonging to any of the following groups:
a) peptides of SEQ ID No. 1 to SEQ ID No. 11;
b) peptides of SEQ ID No. 42 to SEQ ID No. 51 and SEQ ID No. 7.

In an alternative embodiment, the set of overlapping peptides is of the variable Nef antigen of HIV-1. Preferably the set of overlapping peptides consists of peptides belonging to any of the following groups:
a) peptides of SEQ ID No. 12 to SEQ ID No. 26;
b) peptides of SEQ ID No. 27 to SEQ ID No. 41;
c) peptides of SEQ ID No. 12 to SEQ ID No. 41;
d) peptides of SEQ ID No. 52 to SEQ ID No.63 and SEQ ID No. 20, SEQ ID No. 23 and SEQ ID No. 26;
e) peptides of SEQ ID No. 64 to SEQ ID No. 76 and SEQ ID No. 30, SEQ ID No. 31 and SEQ ID No.32;
f) peptides of SEQ ID No. 52 to SEQ ID No. 76 and SEQ ID No. 20, SEQ ID No. 23, SEQ ID No. 26, SEQ ID No. 30, SEQ ID No. 31 and SEQ ID No.32;
g) peptides of SEQ ID No. 77 to SEQ ID No. 86.

It is a further object of the invention the set of overlapping peptides as defined above for use as a medicament, a vaccine or a vaccine adjuvant.

The set of overlapping peptides are used for monitoring CD4 and/or CD8 T cell responses in HIV patients ex vivo, in particular, for the development of immunological diagnostic test or kits. The peptides of the invention can be used for the development of any immunological test that it is based on measuring either the amount of T-cells that are specific for a given antigen or the function of the same cells.

The set of overlapping peptides are used for identifying and/or selecting and/or monitoring a HIV-1 negative population for anti HIV-1 vaccine development; for the detection, in a biological sample, of a population of T cells belonging to any of the following groups:
a) CD4 T cells;
b) CD8 T cells;
c) CD45RA⁺IFNγ⁻ MIP1β⁺ CD8 T cells.

The set of overlapping peptides of the invention can be used also for monitoring T-cell responses in HIV-negative individuals, subjected to preventative anti-HIV vaccine experimentations or in HIV seropositive individual subjected to therapeutic vaccination trials, independently from the need for the identification of the CD45 RA+ MIP1β+ cells.

It is a further object of the invention an isolated population of CD45RA⁺IFNγ⁻ MIP1β⁺ CD8 T cells. Such specific cell population can be advantageously used as a marker for the non-progressive status of HIV-infected subjects, or as immune therapy. As a matter of facts, after sorting and expansion, expanded cells can then be re-infused in HIV infected patients.

The HIV-1 infected subject may be a subject not assuming antiretroviral therapy in which the capacity to control HIV disease and determine the timing of introduction of antiretroviral treatment is to be determined. More preferably the CD45RA⁺ IFNγ⁻ MIP1β⁺ CD8 T cell population is Nef specific.

The CD45RA⁺ IFNγ⁻ MIP1β⁺ CD8 T cell population may be used for measuring the efficacy of an HIV derived antigen vaccine in a vaccinated subject. The vaccinated subject can be HIV seropositive or not.

CD45RA⁺ IFNγ⁻ MIP1β⁺ CD8 T cells are preferably detected using the VOSPD overlapping peptides of the invention. However, the detection of the CD45RA⁺ IFNγ⁻ MIP1β⁺ CD8 T cell can be performed also using the standard 20mers pools or any other antigenic formulation known to those skilled in the art.

Therefore the invention comprises also a method for detecting a CD45RA⁺ IFNγ⁻ MIP1β⁺ CD8 T cell population in a biological sample comprising incubating said sample with a set of peptides belonging to any of the following groups:
a) the set of overlapping peptides according as above defined;
b) standard pools of overlapping peptides;
c) pools of optimal CD8 T-cell epitopes.

The detection of the CD45RA⁺ IFNγ⁻ MIP1β⁺ CD8 T cell population can be performed with assays known to those skilled in the art like, for example, Bioplex, ELISPOT and ELISA. For example, using the ELISPOT technology it is possible to pre-select CD8+CD45RA+ cells directly on the plate, perform the antigenic stimulation and detect simultaneously the production of MIP-1β and IFN-γ.

Preferably, the detection of the CD45RA⁺ IFNγ⁻ MIP1β+ CD8 T cell population is performed with an intracellular cytokine staining and the VOSPD overlapping peptides of the invention..

The detection of the CD45RA⁺ IFNγ⁻ MIP1β⁺ CD8 T cell population can be performed also with a secretion assay. In this way CD45RA⁺ IFNγ⁻ MIP1β⁺ CD8 T cells are still alive after the staining allowing a successive sorting of such a population.

The invention comprises also a kit to detect a CD45RA⁺ IFNγ⁻ MIP1β⁺ CD8 T cell population in a biological sample comprising a set of peptides belonging to any of the following groups:
a) the set of overlapping peptides according as above defined;
b) standard pools of overlapping peptides;
c) pools of optimal CD8 T-cell epitopes.

The kit is used to detect CD45RA⁺ IFNγ⁻ MIP1β⁺ CD8 T cells if found to be specific for any pathological status either upon viral infection (i.e. HIV, HCV, HBV) or tumoral status.

It is another object of the invention a method for the diagnostic of a long-term non-progressive (LTNP) status, or for monitoring the anti-HIV-specific protective CD8 T cell response, in a biological sample from an HIV-1 infected subject, comprising the steps of:
a) detecting the CD45RA⁺IFNγ⁻ MIP1β⁺ CD8 T cell population, and
b) measuring the % thereof with respect to the total CD8 T cells or to the total responding CD8 T cells.

It is another object of the invention a method for measuring the efficacy of an HIV derived antigen vaccine in a biological sample from a vaccinated HIV-1 infected subject, comprising the steps of:
a) detecting the CD45RA⁺IFNγ⁻ MIP1β⁺ CD8 T cell population, and
b) measuring the % thereof with respect to the total CD8 T cells or to the total responding CD8 T cells.

In the present invention the standard pool Tat 15mer comprises:

| | | | |
|---|---|---|---|
| TC27 | 1-15 | MEPVDPRLEPWKHPG | **SEQ ID 87** |
| TC28 | 6-20 | PRLEPWKHPGS**Q**PKT | **SEQ ID 88** |
| TC29 | 11-25 | WKHPGS**Q**PKTACTNC | **SEQ ID 89** |
| TC30 | 16-30 | S**Q**PKTACTNCYCKKC | **SEQ ID 90** |
| TC31 | 21-35 | ACTNCYCKKCCFHCQ | **SEQ ID 91** |
| TC32 | 26-40 | YCKKCCFHCQVCF**I**T | **SEQ ID 92** |
| TC33 | 31-45 | CFHCQVCF**I**TKALGI | **SEQ ID 93** |
| TC34 | 36-50 | VCF**I**TKALGISYGRK | **SEQ ID 94** |
| TC35 | 41-55 | KALGISYGRKKRRQR | **SEQ ID 95** |
| TC36 | 46-60 | SYGRKKRRQRRR**PP**Q | **SEQ ID 96** |
| TC37 | 51-65 | KRRQRRR**PP**Q**G**SQTH | **SEQ ID 97** |
| TC38 | 56-70 | RR**PP**Q**G**SQTHQ**V**SLS | **SEQ ID 98** |
| TC39 | 61-75 | **G**SQTHQ**V**SLSKQP**T**S | **SEQ ID 99** |
| TC40 | 66-80 | Q**V**SLSKQP**T**SQ**S**RGD | **SEQ ID 100** |
| TC41 | 71-85 | KQP**T**SQ**S**RGDPTGPK | **SEQ ID 101** |
| TC42 | 76-90 | Q**S**RGDPTGPKEQKKK | **SEQ ID 102.** |

The pool or set Tat-VOPSD (HIV-1B Tat Bh-10) comprises,

| | | | | |
|---|---|---|---|---|
| Peptide N°1 | MEPVDPRLEPW | 11 mer | | **SEQ ID 1** |
| Peptide N°2 | LEPWKHPGSQPKTACTNCY | 19 mer | (7aa gap) | **SEQ ID 2** |
| Peptide N°3 | GSQPKTACTNCYCKKCCF | 18 mer | (7aa gap) | **SEQ ID 3** |
| Peptide N°4 | ACTNCYCKKCCFHCQVCF | 18 mer | (6aa gap) | **SEQ ID 4** |
| Peptide N°5 | KKCCFHCQVCFITKALGI | 18 mer | (7aa gap) | **SEQ ID 5** |
| Peptide N°6 | QVCFITKALGISYGR | 15 mer | (7aa gap) | **SEQ ID 6** |
| Peptide N°7 | LGISYGRKKRRQRR | 14 mer | (8aa gap) | **SEQ ID 7** |
| Peptide N°8 | KKRRQRRRPPQGSQTHQV | 18 mer | (7aa gap) | **SEQ ID 8** |
| Peptide N°9 | RPPQGSQTHQVSLSKQPT | 18 mer | (7aa gap) | **SEQ ID 9** |
| Peptide N°10 | HQVSLSKQPTSQSRGDPT | 18 mer | (8aa gap) | **SEQ ID 10** |
| Peptide N°11 | KQPTSQSRGDPTGPK | 15 mer | (6aa gap) | **SEQ ID 11;** |

The standard pool Tat 20mer is described in http://www.nibsc.ac.uk/spotlight/aidsreagent/index.html under the reference number EV779.1 to EVA779.8 at page 123, as follows:

| | | |
|---|---|---|
| ARP779.1 | EPVDPRLEPWKHPGSQPKTA | **SEQ ID 123** |
| ARP779.2 | KHPGSQPKTACTTCYCKKCC | **SEQ ID 124** |
| ARP779.3 | CTTCYCKKCCFHCQVCFTTK | **SEQ ID 125** |
| ARP779.4 | FHCQVCFTTKALGISYGRKK | **SEQ ID 126** |
| ARP779.5 | ALGISYGRKKRRQRRRPPQG | **SEQ ID 127** |
| ARP779.6 | RRQRRRPPQGSQTHQVSLSK | **SEQ ID 128** |
| ARP779.7 | SQTHQVSLSKQPTSQPRGD | **SEQ ID 129** |
| ARP779.8 | QPTSQPRGDPTGPKE | **SEQ ID 130.** |

The pool Nef N-ter VOPSD (HIV-1B Nef Bru) comprises,

| | | | | |
|---|---|---|---|---|
| Peptide N°1 | MGGKWSKSSVVGWPTVR | 17 mer | | **SEQ ID 12** |
| Peptide N°2 | SVVGWPTVRERMRRAEPAA | 19 mer | (8aa gap) | **SEQ ID 13** |
| Peptide N°3 | VRERMRRAEPAADGVGAA | 18 mer | (7aa gap) | **SEQ ID 14** |
| Peptide N°4 | AEPAADGVGAASRDLEK | 17 mer | (7aa gap) | **SEQ ID 15** |
| Peptide N°5 | GVGAASRDLEKHGAIT | 16 mer | (6aa gap) | **SEQ ID 16** |
| Peptide N°6 | DLEKHGAITSSNTAATNA | 18 mer | (7aa gap) | **SEQ ID 17** |
| Peptide N°7 | ITSSNTAATNAACAWL | 16 mer | (7aa gap) | **SEQ ID 18** |
| Peptide N°8 | ATNAACAWLEAQEEEEV | 17 mer | (7aa gap) | **SEQ ID 19** |
| Peptide N° 9 | CAWLEAQEEEE | 11 mer | (5aa gap) | **SEQ ID 20** |
| Peptide N°10 | QEEEEVGFPVTPQVPLR | 17 mer | (5aa gap) | **SEQ ID 21** |
| Peptide N°11 | VGFPVTPQVPLRPMTYK | 17 mer | (5aa gap) | **SEQ ID 22** |
| Peptide N°12 | PQVPLRPMTY | 10 mer | (6aa gap) | **SEQ ID 23** |
| Peptide N°13 | RPMTYKAAVDLSHFLK | 16 mer | (5aa gap) | **SEQ ID 24** |
| Peptide N°14 | AVDLSHFLKEKGGLEGL | 17 mer | (7aa gap) | **SEQ ID 25** |
| Peptide N°15 | FLKEKGGLEGLI | 12 mer | (6aa gap) | **SEQ ID 26.** |

The pool Nef C-ter VOPSD (HIV-1B Nef Bru) comprises,

| | | | | |
|---|---|---|---|---|
| Peptide N°16 | LEGLIHSQRRQDILDLWIY | 19 mer | (7aa gap) | **SEQ ID 27** |
| Peptide N°17 | QRRQDILDLWIYHTQGY | 17 mer | (7aa gap) | **SEQ ID 28** |
| Peptide N°18 | LDLWIYHTQGYFPDWQNY | 18 mer | (6aa gap) | **SEQ ID 29** |
| Peptide N°19 | YHTQGYFPDWQNYT | 14 mer | (6aa gap) | **SEQ ID 30** |
| Peptide N°20 | YFPDWQNYTPGPGVRY | 16 mer | (5aa gap) | **SEQ ID 31** |
| Peptide N°21 | QNYTPGPGVRYPLTFGW | 17 mer | (5aa gap) | **SEQ ID 32** |
| Peptide N°22 | GPGVRYPLTFGWCYKL | 16 mer | (5aa gap) | **SEQ ID 33** |
| Peptide N°23 | TFGWCYKLVPVEPDKVEEA | 19 mer | (8aa gap) | **SEQ ID 34** |
| Peptide N°24 | VPVEPDKVEEANKGENTSL | 19 mer | (8aa gap) | **SEQ ID 35** |
| Peptide N°25 | KVEEANKGENTSLLHPV | 17 mer | (6aa gap) | **SEQ ID 36** |
| Peptide N°26 | ENTSLLHPVSLHGMDDPER | 19 mer | (8aa gap) | **SEQ ID 37** |
| Peptide N°27 | PVSLHGMDDPEREVLEWR | 18 mer | (7aa gap) | **SEQ ID 38** |
| Peptide N°28 | DDPEREVLEWRFDSRLAF | 18 mer | (7aa gap) | **SEQ ID 39** |
| Peptide N°29 | VLEWRFDSRLAFHHVAREL | 19 mer | (6aa gap) | **SEQ ID 40** |
| Peptide N°30 | DSRLAFHHVARELHPEYF | 18 mer | (6aa gap) | **SEQ ID 41.** |

The standard pool Nef N-ter 20mers is described in http://www.nibsc.ac.uk/spotlight/aidsreagent/index.html under the reference number ARP7074.1 to ARP7074.10 at page 121, as follows:

| | | |
|---|---|---|
| ARP7074.1 | GGKWSKSSVVGWPTVRERMR | **SEQ ID 103** |
| ARP7074.2 | GWPTVRERMRRAEPAADGVG | **SEQ ID 104** |
| ARP7074.3 | RAEPAADGVGAASRDLEKHG | **SEQ ID 105** |
| ARP7074.4 | AASRDLEKHGAITSSNTAAT | **SEQ ID 106** |
| ARP7074.5 | AITSSNTAATNAACAWLEAQ | **SEQ ID 107** |
| ARP7074.6 | NAACAWLEAQEEEEVGFPVT | **SEQ ID 108** |
| ARP7074.7 | EEEEVGFPVTPQVPLRPMTY | **SEQ ID 109** |
| ARP7074.8 | PQVPLRPMTYKAAVDLSHFL | **SEQ ID 110** |
| ARP7074.9 | KAAVDLSHFLKEKGGLEGLI | **SEQ ID 111** |
| ARP7074.10 | KEKGGLEGLIHSQRRQDILD | **SEQ ID 112** |

The standard pool Nef C-ter 20mers is described in http://www.nibsc.ac.uk/spotlight/aidsreagent/index.html under the reference number ARP7074.11 to ARP7074.20 at page 121 , as follows:

| | | |
|---|---|---|
| ARP7074.11 | HSQRRQDILDLWIYHTQGYF | **SEQ ID 113** |
| ARP7074.12 | LWIYHTQGYFPDWQNYTPGP | **SEQ ID 114** |
| ARP7074.13 | PDWQNYTPGPGVRYPLTFGW | **SEQ ID 115** |
| ARP7074.14 | GVRYPLTFGWCYKLVPVEPD | **SEQ ID 116** |
| ARP7074.15 | CYKLVPVEPDKVEEANKGEN | **SEQ ID 117** |
| ARP7074.16 | KVEEANKGENTSLLHPVSLH | **SEQ ID 118** |
| ARP7074.17 | TSLLHPVSLHGMDDPEREVL | **SEQ ID 119** |
| ARP7074.18 | GMDDPEREVLEWRFDSRLAF | **SEQ ID 120** |
| ARP7074.19 | EWRFDSRLAFHHVARELHPE | **SEQ ID 121** |
| ARP7074.20 | HHVARELHPEYFKNC | **SEQ ID 122** |

The pool Tat C VOPSD (HIV-1B Tat consensus C) comprises:

| | | | | |
|---|---|---|---|---|
| Peptide N°1 | MEPVDPNLEPW | 11 mer | **SEQ ID 42** | |
| Peptide N°2 | LEPWNHPGSQPKTACNTCY | 19 mer | (7aa gap) | **SEQ ID 43** |
| Peptide N°3 | GSQPKTACNTCYCKKCSY | 18 mer | (7aa gap) | **SEQ ID 44** |
| Peptide N°4 | ACNTCYCKKCSYHCLVCF | 18 mer | (6aa gap) | **SEQ ID 45** |
| Peptide N°5 | KKCSYHCLVCFQTKGLGI | 18 mer | (7aa gap) | **SEQ ID 46** |
| Peptide N°6 | LVCFITKGLGISYGR | 15 mer | (7aa gap) | **SEQ ID 47** |
| Peptide N°7 | KKRRQRRSAPPSSEDHQN | 18 mer | (7aa gap) | **SEQ ID 48** |
| Peptide N°8 | SAPPSSEDHQNPISKQPL | 18 mer | (7aa gap) | **SEQ ID 49** |
| Peptide N°9 | HQNPISKQPLPRTLGDPT | 18 mer | (8aa gap) | **SEQ ID 50** |
| Peptide N°10 | KQPLPRTLGDPTGSE | 15 mer | (6aa gap) | **SEQ ID 51** |
| and the Peptide LGISYGRKKRRQRR,**SEQID7**; | | | | |

The pool Nef C N-ter VOPSD (HIV-1 Nef Consensus C) comprises:

| | | | | |
|---|---|---|---|---|
| Peptide N°1 | MGGKWSKCSIVGWPAVR | 17 mer | **SEQ ID 52** | |
| Peptide N°2 | SIVGWPAVRERMRRTEPAA | 19 mer | (8aa gap) | **SEQ ID 53** |
| Peptide N°3 | VRERMRRTEPAAEGVGAA | 18 mer | (7aa gap) | **SEQ ID 54** |
| Peptide N°4 | TEPAAEGVGAASQDLDK | 17 mer | (7aa gap) | **SEQ ID 55** |
| Peptide N°5 | GVGAASQDLDKHGALT | 16 mer | (6aa gap) | **SEQ ID 56** |
| Peptide N°6 | DLDKHGALTSSNTAANNA | 18 mer | (7aa gap) | **SEQ ID 57** |
| Peptide N°7 | LTSSNTAANNADCAWL | 16 mer | (7aa gap) | **SEQ ID 58** |
| Peptide N°8 | ANNADCAWLEAQEEEEEV | 18 mer | (7aa gap) | **SEQ ID 59** |
| Peptide N°9 | CAWLEAQEEEE | 11 mer | (5aa gap) | **SEQ ID 20** |
| Peptide N°10 | QEEEEEVGFPVRPQVPLR | 18 mer | (6aa gap) | **SEQ ID 60** |
| Peptide N°11 | VGFPVRPQVPLRPMTYK | 17 mer | (5aa gap) | **SEQ ID 61** |
| Peptide N°12 | PQVPLRPMTY | 10 mer | (6aa gap) | **SEQ ID 23** |
| Peptide N°13 | RPMTYKAAFDLSFFLK | 16 mer | (5aa gap) | **SEQ ID 62** |
| Peptide N°14 | AFDLSFFLKEKGGLEGL | 17 mer | (7aa gap) | **SEQ ID 63** |
| Peptide N°15 | FLKEKGGLEGLI | 12 mer | (6aa gap) | **SEQ ID 26.** |

The pool Nef C C-ter VOPSD (HIV-1 Nef Consensus C) comprises:

| | | | | |
|---|---|---|---|---|
| Peptide N°16 | LEGLIYSKKRQEILDLWVY | 19 mer | (7aa gap) | **SEQ ID 64** |
| Peptide N°17 | KKRQEILDLWVYHTQGY | 17 mer | (7aa gap) | **SEQ ID 65** |
| Peptide N°18 | LDLWVYHTQGYFPDWQNY | 18 mer | (6aa gap) | **SEQ ID 66** |
| Peptide N°19 | YHTQGYFPDWQNYT | 14 mer | (6aa gap) | **SEQ ID 30** |
| Peptide N°20 | YFPDWQNYTPGPGVRY | 16 mer | (5aa gap) | **SEQ ID 31** |
| Peptide N°21 | QNYTPGPGVRYPLTFGW | 17 mer | (5aa gap) | **SEQ ID 32** |
| Peptide N°22 | GPGVRYPLTFGWCFKL | 16 mer | (5aa gap) | **SEQ ID 67** |
| Peptide N°23 | TFGWCFKLVPVDPREVEEA | 19 mer | (8aa gap) | **SEQ ID 68** |
| Peptide N°24 | VPVDPREVEEANEGENNCL | 19 mer | (8aa gap) | **SEQ ID 69** |
| Peptide N°25 | EVEEANEGENNCLLHPM | 17 mer | (6aa gap) | **SEQ ID 70** |
| Peptide N°26 | ENNCLLHPMSQHGMEDEHR | 19 mer | (8aa gap) | **SEQ ID 71** |
| Peptide N°27 | PMSQHGMEDEHREVLKWK | 18 mer | (7aa gap) | **SEQ ID 72** |
| Peptide N°28 | EDEHREVLKWKFDSHLAR | 18 mer | (7aa gap) | **SEQ ID 73** |
| Peptide N°29 | VLKWKFDSHLARRHMAREL | 19 mer | (6aa gap) | **SEQ ID 74** |
| Peptide N°30 | DSHLARRHMARELHPEYY | 18 mer | (6aa gap) | **SEQ ID 75** |
| Peptide N°31 | RHMARELHPEYYKDC; | 15 mer | (6aa gap) | **SEQ ID 76;** |

the pool Nef C SA VOPSD (HIV-1C South African Nef Consensus) comprises,

| | | | |
|---|---|---|---|
| Peptide N°1 | YHTQGFFPDWQNYT | 14 mer | **SEQ ID 77** |
| Peptide N°2 | FFPDWQNYTPGPGVRY | 16 mer | **SEQ ID 78** |
| Peptide N°3 | DLDKHGALTSSNTAHNNA | 18 mer | **SEQ ID 79** |
| Peptide N°4 | LTSSNTAHNNADCAWL | 16 mer | **SEQ ID 80** |
| Peptide N°5 | HNNADCAWLEAQEEEEEV | 18 mer | **SEQ ID 81** |
| Peptide N°6 | LDLWVYHTQGFFPDWQNY | 18 mer | **SEQ ID 82** |
| Peptide N°7 | PMSQHGMEDEDREVLKWK | 18 mer | **SEQ ID 83** |
| Peptide N°8 | EDEDREVLKWKFDSSLAR | 18 mer | **SEQ ID 84** |
| Peptide N°9 | VLKWKFDSSLARRHMAREL | 19 mer | **SEQ ID 85** |
| Peptide N°10 | DSSLARRHMARELHPEYY | 18 mer | **SEQ ID 86.** |

The present invention shall be disclosed in detail also by means of non limiting examples referring to the following figures.

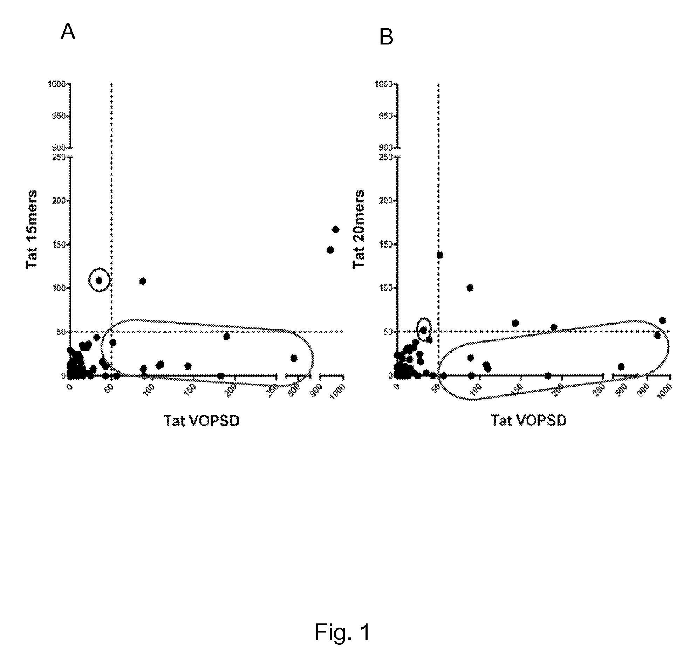

### Figure 1. Comparison between peptide pools encoding the Tat antigen.

Comparison between Tat VOPSD and Tat 15mers responses (Figure 1A) and between Tat VOPSD and Tat 20mers (Figure 1B). The circle individuates the two responses not detected by the Tat VOPSD pool. Ellipses individuate the responses detected only using the Tat VOPSD peptide pools. The data reported on the x and y axes are expressed as SFU/1,000,000 cells.

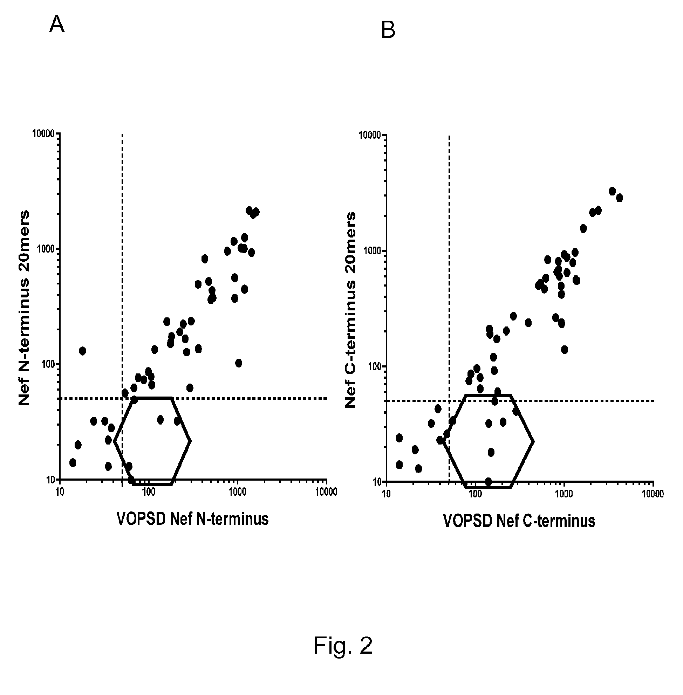

### Figure 2. Comparison between peptide pools encoding the Nef antigen.

Comparison between VOPSD peptide pools and standard pools. (2A) Nef N-terminus VOPSD encoding the protein N-terminus vs Nef N-ter 20mers. (2B) Nef C-terminus VOPSD encoding the protein C-terminus vs. Nef C-ter 20mers C-terminus. Hexagons individuate the responses detected only using the VOPSD peptide pools. The data reported on the x and y axes are expressed as SFU/1,000,000 cells.

### Figure 3. Comparison between CD8 and CD4 T cell responses obtained using different peptide pools encoding the Nef antigen.

Comparison between the CD8 responses (A and B) and the CD4 responses (C and D) obtained using the peptide pools (VOPSD N-terminus and Nef N-terminus 20mers) encompassing the protein N-terminus (A and C) or C-terminus (VOPSD C-terminus and Nef N-terminus 20mers) (B and D).

### Figure 4. Comparison between VOPSD and 20mers peptide pools encoding the HIV-1 antigens Tat and Nef.

Panel 4A: Recognition of the Tat VOPSD (grey line with filled diamond) and its 20mer standard homolog (Tat 20mer) (black line with filled circle) by PBMC derived from patient HSR-006.

Panel 4B: Recognition of VOPSD Nef N-terminus peptide pool and its standard 20mer homolog (NefN-terminus 20mers) by patient HSR-018. Simbols and line colors are reported as above described.

The dotted line individuates the negative cut-off of the assay in both panels.

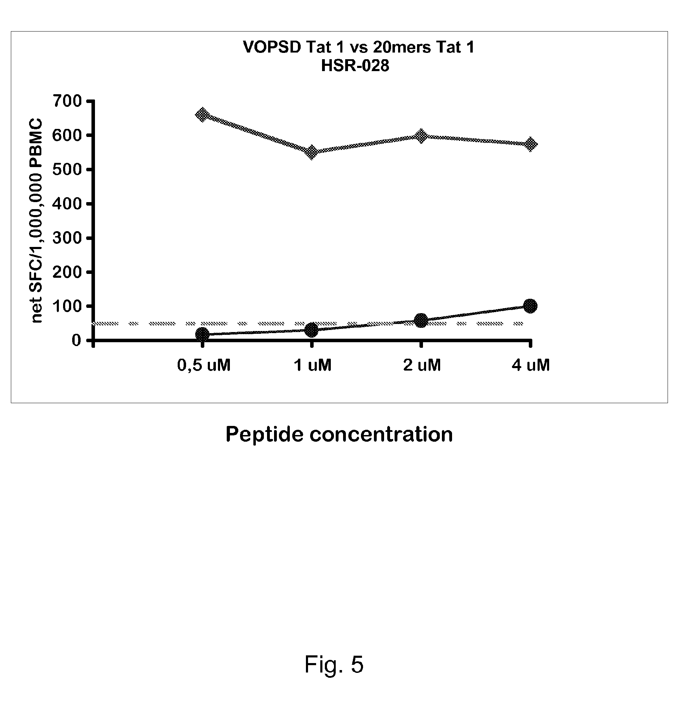

### Figure 5. single peptide Recognition of homologs peptides belonging to the Tat VOPSD and the Tat 20mers peptide sets.

Recognition of the VOPSD Tat peptide 1 (greyline with filled diamond) and its standar Tat 20mer homolog (black line with filled circle) by PBMC derived from patient HSR-028.

The dotted line individuates the negative cut-off of the assay.

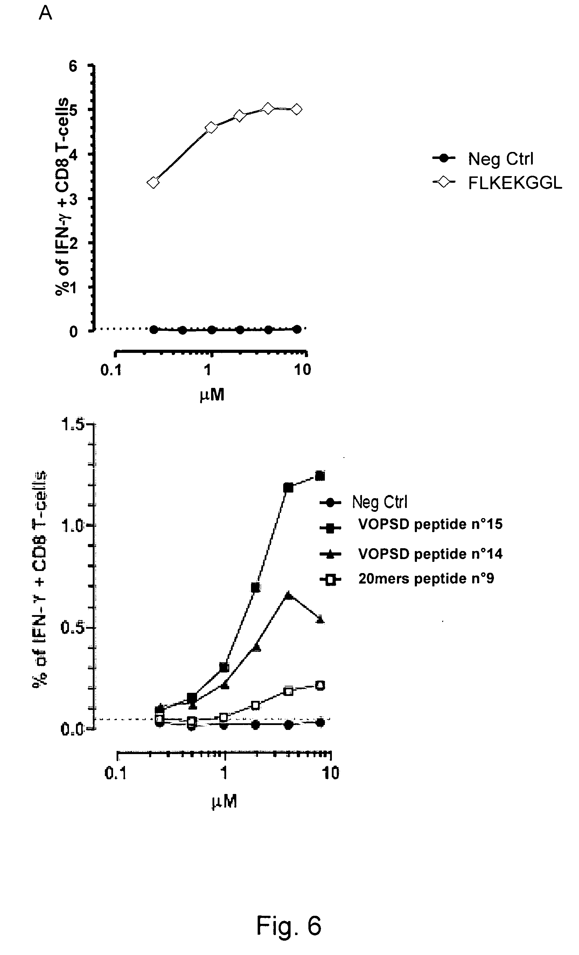

### Figure 6. CD8 T cells recognition of the minimal epitope FLKEKGGL present in the Nef specific overlapping set of peptides.

Panel 6A: recognition of the minimal epitope Class I B 08 restricted FLKEKGGL (SEQ ID 131) by CD8 T-cell belonging to patient V4 PBMC from subject V4 were stimulated with the FLKEKGGL peptide (grey diamond) or with an equivalent volume of DMSO (black circles), before staining for the detection of intracellular IFN-γ.

Panel 6B: recognition of the overlapping peptides containing the FLKEKGGL epitope. PBMC were stimulated with two Nef derived peptide from the Nef N-ter VOPSD set (FLKEKGGLEGLI, SEQ ID No. 26, black squares; AVDLSHFLKEKGGLEGL, SEQ ID No. 25 black triangles) and one peptide from the classical Nef 20mer set (KAAVDLSHFLKEKGGLEGLI, SEQ ID No. 111, open squares). As negative control, PBMCs were stimulated with medium alone containing an equal volume of DMSO (black circles).

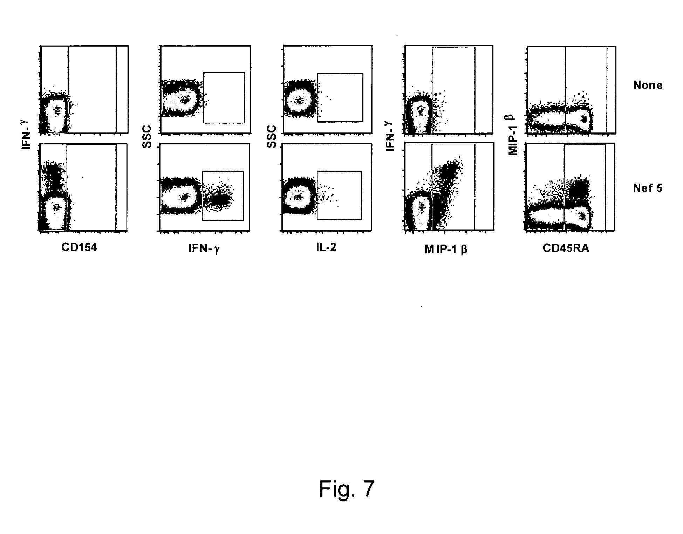

**Figure 7****. Gating strategy.** The pseudo-colour dot plots show an example of gating strategy applied on CD8+ CD3+ living lymphocytes. A mock stimulated (none) and Nef C-termVOPSD (Nef 5) stimulated samples are shown.

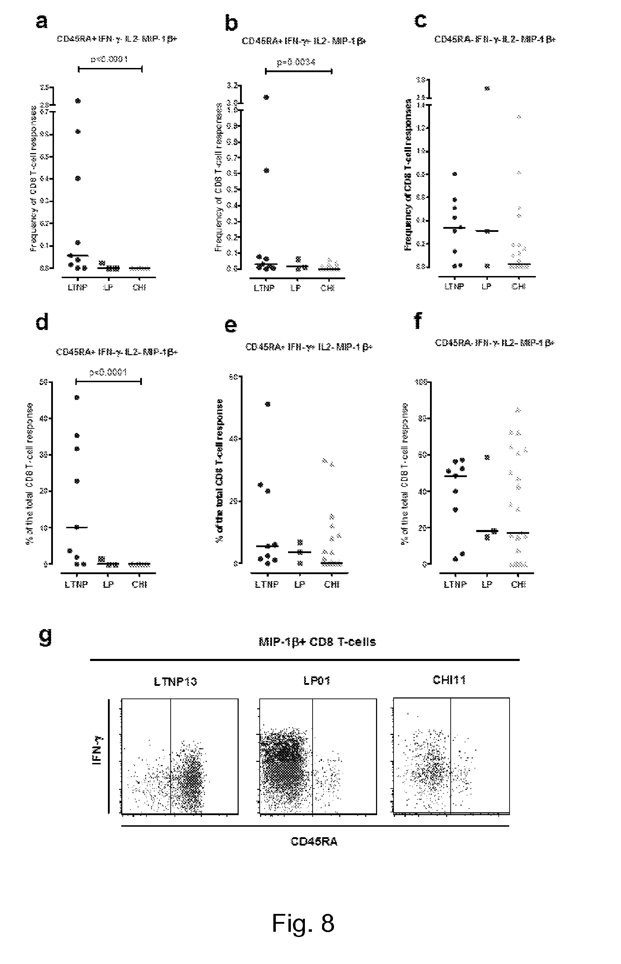

**Figure 8****. Single data representation for the interesting populations and representative dot plots.** Frequencies of CD8 T-cell responses (A, B and C) and % of the total response (D, E and F) are shown for the population discussed in the text: CD45RA+ IFN-γ- IL-2- MIP-1β+ (A and D), CD45RA+ IFN-γ+ IL-2- MIP-1β+ (B and E) and CD45RA- IFN-γ- IL-2- MIP-1β+ (C and F). Each point represents one patient. Medians are shown for each cohort. In (G) representative dot plots from a long term non progressor (LTNP), a late progressor (LP) and a chronically infected individual (CHI) are shown. Depicted events were previously gated on MIP-1β+ CD8 T-cells. The vertical line in each dot plot separates CD45RA positive and negative cells. IFN-γ+ cells are depicted in blue.

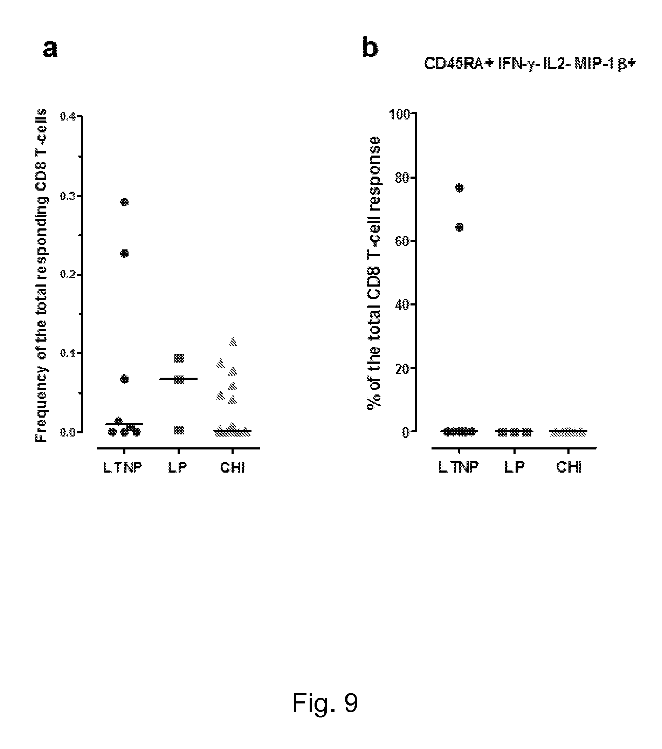

**Figure 9****. Single data representation for the percentage of the Tat-specific CD45RA+ IFN-γ- IL-2- MIP-1β+ responding CD8 T-cells.** Each point represents one patient. Medians are shown for each cohort.

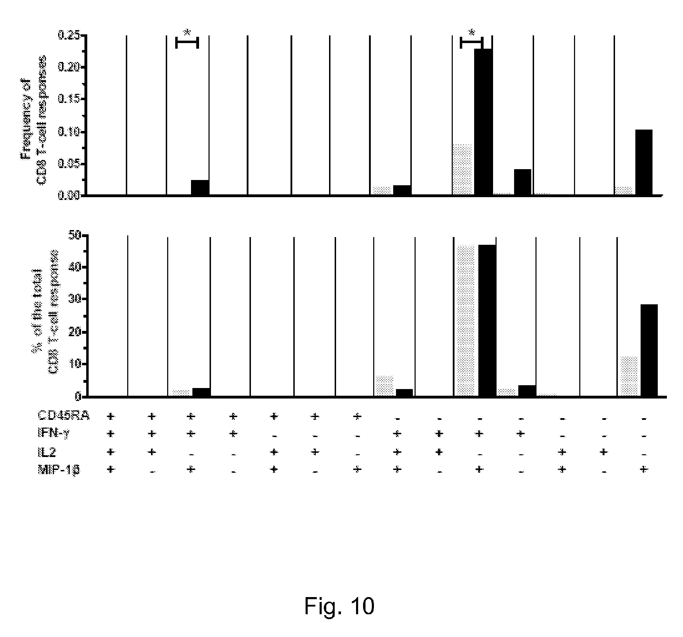

**Figure 10****. Characterization of the Nef-specific CD8 T-cell responses before and after therapy interruption.** Absolute and relative frequencies of the Nef-specific response patterns for CD8 T-cells. The histogram plot shows the median frequency of the responding T-cells. Responses to the pools Nef N-term VOPSD and Nef C-term VOPSD are summed together to obtain the response to the entire Nef antigen. Asterisks indicate significant differences. The grey bars represent values before therapy interruption, the black bars represent the values after therapy interruption.

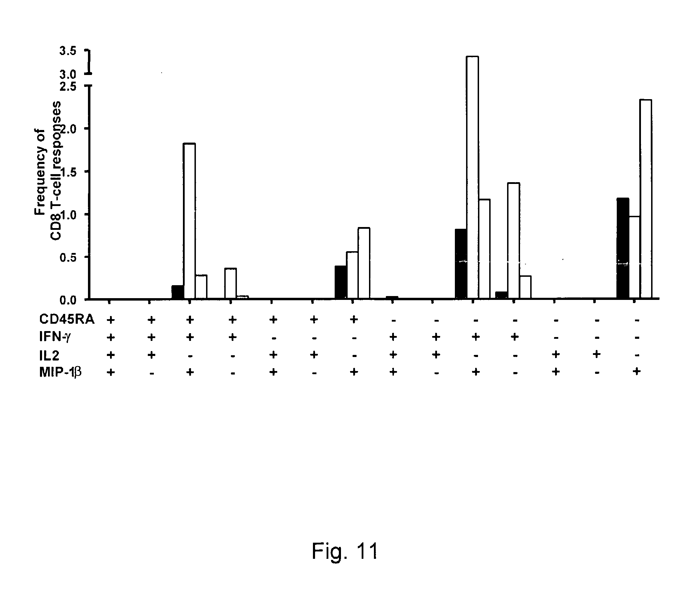

**Figure 11****. Comparison between VOPSD, a pool of Nef derived optimal CD8 T-cell epitopes and the standard 20 mer in detecting the CD45RA+ IFN-γ- IL-2- MIP-1β+ CD8 T-cell population.** Absolute frequencies of Nef-specific response patterns for CD8 T-cells are shown. The histogram plot shows the frequency of the responding T-cells. Responses detected with the pools Nef N-term VOPSD and Nef C-term VOPSD (white bars), the optimal pool (grey bar) and with the standard 20mer (black bars) are shown.

### METHODS

### Patients

Three cohorts of HIV-1 seropositive individuals and two cohorts of HIV-seronegative healthy blood donors were enrolled for the study (Table I).

**Table I: Summary of parameters for HIV-1 seropositive cohorts enrolled for the study**

| Cohort | CD4 T cell count | | HIV plasma load | | Years of known seropositi vity | ART |
|---|---|---|---|---|---|---|
| | median | 1° -3° quartile | median | 1° - 3°quartile | | |
| 1 (n = 75) | 549 | 464-838 | 10685 | 860-28423 | Weeks-22 yrs | |
| 2 (n = 14) | 417 | 375-593 | 50 | 50-472 | | |
| 3 (n = 9) | 502 | 441-775 | 1083 | 351-5377 | > 13 yrs | |
| | 395 | | 50 | | > 20 yrs | |
| 3 (n = 3) | 488 | 351-698 | 50 | | > 2 yrs | ART for > 2 yrs |
| | | | | | | |
| 3 (n = 23) | | | | | | |

In the largest cohort, used for the IFN-γ based ELISPOT, the authors enrolled 76 HIV-1 infected individuals with variable CD4 T cell counts (median 549; 1°-3° quartile 464 and 838 CD4/µl) and HIV-1 plasma load (median 10,685; 1°-3° quartile 860 and 28,423 genome equivalents/ml), as well as for length of the HIV-1 infection (from few weeks to over 22 years), route of infection and antiretroviral therapy (ART). Patients HSR 006, 018 and 028 belonged to this cohort. The smallest cohort (14 individuals), used for the immunocytofluorimetric assay, was composed by HIV-seropositive individuals with a CD4 T-cell count > 300 cells/µl (median 417; 1°-3° quartile 375 and 593) and low HIV-1 plasma load (median 50; 1°-3° quartile 50 and 472 genome equivalents/ml). Patient V4 belonged to this cohort. In the third one the authors enrolled a total of 35 HIV-1 infected individuals belonging to three different groups of patients. The first group was composed by 9 long term non progressors (LTNP) HIV-seropositive patients for more than 13 years, median CD4 T cell count of 502 cell/µl (1°-3° quartile 441 and 775) and median HIV-1 plasma load of 1083 genome equivalents/ml (1°-3° quartile 351 and 5377). The second group was composed by 3 late progressors (LP) patients that were HIV-seropositive for >20 years, They progressed to AIDS during the last year of observation and one (LP01) was not yet ART-treated when the blood sample was withdrawn. The third group included 23 chronically infected individuals (CHI) HIV-seropositive and under ART for >2 years The median CD4 T-cell count was 488 cell/µl (1°-3° quartile 351 and 698) and viral load was undetectable in all but three subject (CHI-011 3,362 genome equivalents/ml; CHI-012: 36,600 genome equivalents/ml; CHI-3V: 13,965 genome equivalents/ml).

Six CHI patients were tested again after an interruption of highly active antiretroviral therapy (HAART) for 2-4 weeks; the therapy was reintroduced when the viral load was higher than 100,000 genome equivalent /ml.

Finally, to assess the background responses generated by cross-reactive T-cells a cohort of 75 HIV-seronegative healthy blood donors (HBD) was investigated using the ELISPOT technique, whereas a second small cohort of 5 HBD was used for the immunocytofluorimetric assay.

### Antigens

Overlapping sets of peptides covering the HIV-1 encoded Tat (BH-10 strain; aa 1- 86 Gene bank accession number M15654) and Nef antigen (Bru strain; aa 1-205 gene bank accession number K02013 ) were used. For the Tat antigen both standard strategies of protein scanning design were utilized building-up two pools of peptides composed by either 15mers overlapped by 10 aa residues (designated Tat15mer) or 20mers overlapped by 10aa residues (designated Tat20mer). Only one peptide set formed by 20mers overlapped by 10aa residues was used for the Nef antigen (the set is composed by peptides Nef N-ter 20mers and Nef C-ter 20mers) to validate the new overlapping peptide antigen scanning strategy.

A peptide pool composed by 32 CD8 immunodominant restricted epitopes derived from Influenza, citomegalo and Ebstain-Barr viruses (FEC pool) was used as HIV-1-unrelated positive control for monitoring the level of T-cell responses.
the Fec pool is described in http://www.nibsc.ac.uk/spotlight/aidsreagent/index.html under the reference number ARP7099 at page 135 .

### Ex vivo ELISPOT assay for single-cell IFN-γ release

Peripheral blood mononuclear cells (PBMC) were seeded in duplicate at 2X10⁵ cells/well in 96-well plates (MAIPS4510; Millipore, Bedford, Mass.) precoated with anti-IFN-γ capture monoclonal antibody (B-B1; Diaclone, Besançon, France) and stimulated with the different peptide pools at the fixed concentration of 3 µg/ml of each peptide for 18 h at 37°C in air plus 5% CO2. Biotinylated anti-IFN-γ detection monoclonal antibody (B-G1; Diaclone) was added for 2 h, followed by the addition of streptavidin-alkaline phosphatase conjugate (Amersham Pharmacia Biotech Europe GmbH, Freiburg, Germany) for 1 h. A chromogenic substrate (nitroblue tetrazolium-BCIP [5-bromo-4-chloro-3-indolylphosphate]; Sigma, St. Louis, Mo.) was added for 10 min. The individual spots were counted by using an Automated ELISA-Spot Assay Video Analysis System, Eli-Scan with the software Eli.Analyse V4.2 (A.EL.VIS, Hannover, Germany).

The responses were empirically scored as positive if the test wells contained a mean number of spot-forming cells (SFC) higher than the mean value plus two standard deviations in negative control wells, and if the number of SFC per million PBMC in stimulated wells (subtracted of the values of negative control wells) was ≥50. PBMC in medium alone were used to establish the negative control background, that had to be not higher than 50 SFC per million of cells (10 SFC/ well). PBMC stimulated with phytohemagglutinin (PHA-P; Sigma) at 5 mg/ml or the FEC peptide pool containing 1,5 µg/ml of each peptide were used as positive controls.

### Intracellular cytokine staining

After thawing, PBMC were washed trice at room temperature in complete cell culture medium and plated in round bottom 96 wells/plates. All the incubations were carried out in round bottom 96 wells/plates to allow high throughput processing of the probes. 10⁶ PBMC/well were incubated with 1.3 µg/ml of antibody to CD28 and with 1.3 µg/ml of antibody to CD49d (Becton Dickinson, Heidelberg, Germany) together with defined peptide pools. For each individual, a sample without peptides was included to calculate the background staining. Following one-hour incubation, 10 µg/ml of Brefeldin A were added to the cell suspension and the incubation carried out for additional 4 hours. Stimulated cells were then resuspended in Stain Buffer (0,2% BSA, 0,09% Na Azide in DPBS; Becton Dickinson) and incubated with the photoreactive fluorescent label ethidium monoazide (EMA; Molecular Probes, Leiden, The Netherlands) used as viability dye. After washing, cells were fixed and permeabilized using the BD Cytofix/Cytoperm™ Kit, (Becton Dickinson). Then, the following fluorochrome-conjugated antibodies were added to the cell suspension: CD3-AmCyan, CD4-PerCP, CD45RA-PECy7, CD154-FITC, IFNγ-A1700, IL2-APC and MIP1β-PE from Becton Dickinson and CD8-PacB from DAKO (Hamburg, Germany). Incubation was carried out on ice for 30 min and after washing, cells were acquired using an LSRII flow cytometer (Becton Dickinson) equipped with a high throughput system. Sample analysis was performed using FlowJo version 8.5.3. Lymphocytes were gated on a forward scatter area versus side scatter area pseudo-color dot plot and dead cells removed according to EMA staining. Events were gated on CD3+ events versus IFN-γ, IL-2, MIP-1β and CD154 to account for down-regulation. Then, CD3+ events were combined together using the Boolean operator "Or". The same procedure was used to successively gate CD8+ events. CD4+ events were excluded before creating a gate for each function or phenotype as shown in Figure 7.

Then, the authors created a series of Boolean gates that represent all the combination of the gate shown in Figure 7.

### Data analysis

Since background levels varied between subpopulations, i.e. CD154 staining showed a higher background than IFN-γ and the combination of three or more functions had extremely low background, the authors calculated a threshold level for each subpopulation. Following background subtraction, the threshold level was calculated using the 90 percentile of the negative values. Then all the resulting negative values were set to zero and all values > 0 were considered as positive responses. SPICE version 4.1.5 was used for threshold calculation and graphical representation of the data. Statistical analysis was performed using SAS Version 9.1 and GraphPad Prism version 5.01. To account for multiple comparisons, the authors performed the Kruskal Wallis test before pair-wise comparisons by the Wilcoxon test. Statistical comparisons were considered moderately significant with a p value < 0.05 (*) significant with a p value <0.01 (**) and highly significant with a p value < 0.001 (***).

### RESULTS

### Peptide design

The new approach of peptide design combines the empirical-based approach with database information such as consensus antigen sequences belonging to different HIV-1 clades, immunodominat antigenic region and optimal epitope so far described.

As a first step the authors have aligned the protein sequence of candidate Tat (derived from the BH-10 strain belonging to clade B viruses) and Nef vaccine antigens (derived from the Bru strain belonging to clade B viruses) with the consensus clade C sequences of the homologous antigen, as presented below (Scheme 1, HIV-1B: SEQ ID No. 132, NEF-SA-CON-C: SEQ ID No. 133, HIV-1C: SEQ ID No. 134).

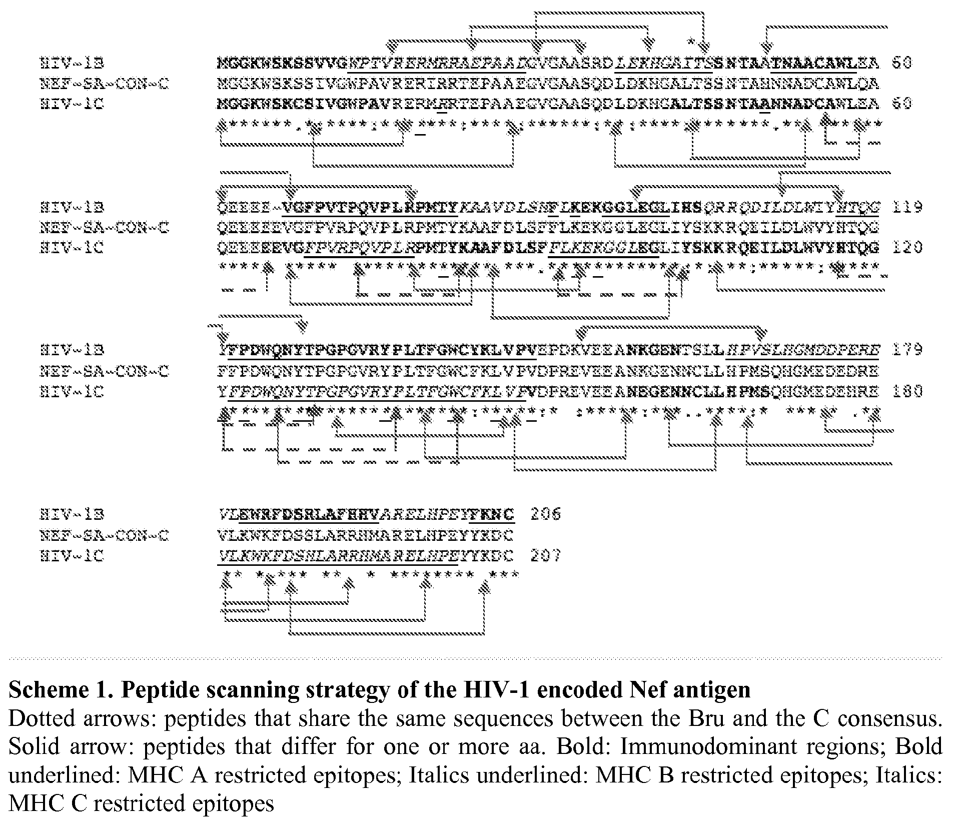

Secondly, the authors have mapped within these sequences all the reported immuno-dominant regions and optimal epitopes so far described. Thirdly, they have individuated conserved regions longer than 10 aa residues that were covered by a specific set of peptides (common peptides), completing the overlapping peptide scanning of both antigens with a set of peptides which contain all the protein fragment that differ for one or more amino acidic residues between the vaccine candidate and the consensus sequences. All the derived peptides were biased at the C-terminus avoiding seven amino acidic residues that were never found among optimal CTL epitopes and class I binding motifs (Asn, Asp, Gln, Glu, Gly, His, Ser). Moreover, the overlapping peptides were design placing the known optimal epitopes right in their N- or C-terminus of the longer scanning peptides.

In order to fit all of the above-mentioned design features, the derived peptides had variable length (from 10 to 19 aa residues) and degree of overlap (with a gap not larger than 8 aa).

Moreover, degree of overlap and overall peptide length were tuned accordingly to the frequency of known T-cell epitopes (higher overlap and shorter peptide length in epitope-rich regions and, conversely, wider not-overlapping fragment and maximal peptide length in epitope- poor regions). In addition, when multiple epitopes partially overlapped, the length of the peptide was chosen in order to fit either the higher number of epitopes or the epitope described for the most frequent Class I HLA allele, Moreover, we also considered, in the case of multiple partially overlapping epitopes, placing the ones that do not fit properly in the C-terminus of a given scanning peptides at the N-terminus of a different scanning peptide, Therefore, since variability in peptide length and degree of overlap is a distinctive novel feature of this peptide design strategy, the the newly generated HIV-1 derived peptide sets were denominated Tat VOPSD, Nef N-ter VOPSD, Nef C-ter VOPSD, TatC VOPSD, NefC N-ter VOPSD, NefC C-ter VOPSD.

### Assessment of background responses on HIV-seronegative individuals

A cohort of 75 HIV-seronegative individuals with different ethnical and geographical origin was employed to measure the level of background T-cell responses generated by each peptide set. As shown in Table II, the IFN-γ elispot assay used showed an extremely low level of background signal (median 5 SFU/1,000,000 cells; 1-3 quartile: 2,5 and 10 SFU/1,000,000 cells) falling well below the maximum accepted threshold for negative controls (<50 SFU/1,000,000 cells; <10 SFU/well).

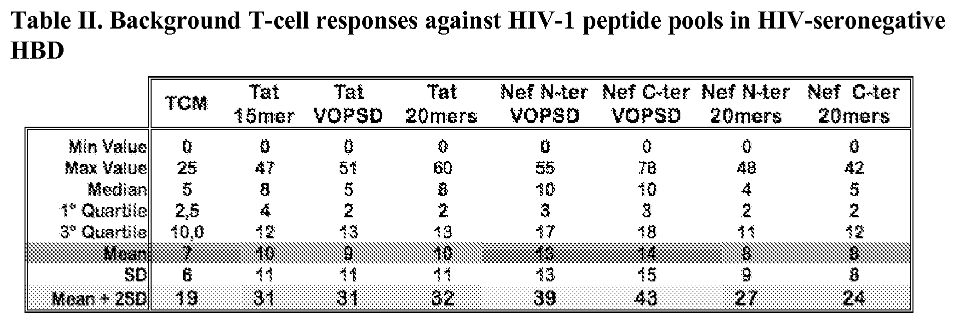

All the Tat specific peptide pools showed a negligible background of cross-reactive responses with both median and mean values not significantly different from the ones measured on negative controls (Table II). VOPSD Nef-specific peptide sets showed a higher background as compared to their homolog 20mers peptide sets or the negative control. However, also in this case both median, mean and mean + 2 deviation standard values of SFU were well below the accepted threshold for negative controls.

### Comparison between distinct sets of Tat specific overlapping peptides for the detection of IFN-gamma secreting cells in HIV-infected individuals

Next, the authors evaluated in a cohorts of 76 HIV-infected patients heterogeneous for CD4 T cell counts, HIV-1-load, time and route of infection, the number of Tat specific T-cell responses detected by each set of overlapping peptides. Fifteen out of the seventy-six individuals recognized Tat specific peptides pools (20%). Interestingly, 13/15 responses were detected using the new peptide VOPSD set, whereas only 4/15 and 6/15 of the total responses were detected using the peptide pools composed by classical Tat 15mers and Tat 20mers peptide pools respectively (Figure 1 A-B). Moreover, this peptide set revealed 3/4 and 5/6 responses detected with the other two pools respectively, and in 2/3 and in 3/5 cases these responses were significantly higher when the VOPSD pool was used (Figure 1A-B).

### Comparison between distinct sets of Nef specific overlapping peptides for the detection of IFN-gamma secreting cells in HIV-infected individuals

The same cohort of HIV-seropositive individuals was investigated to directly compare the ability of Nef specific VOPSD set against a peptide set composed by 20mers with a fix overlap of 10aa in detecting Nef-specific responses. Two pools for each peptide set were prepared: the first two covered the N-terminus and first half of the protein (aa 1-101 for the Nef N-ter VOPSD set and aa 1-110 for the Nef N-ter 20mers peptide set) whereas the other two covered the C-terminus and the other half of the protein (aa 96- 205 and aa 100-205 for Nef C-ter VOPSD and Nef C-ter 20mers, respectively).

In total 95 Nef specific T-cell responses were measured in 59/76 (78%) patients using the two VOPSD pools whereas only 81 responses in 51/76 (67%) patients were detected using the 20mers pools (Figure 2A-B). Interestingly, almost all the responses detected with the 20mers pools (80/81) were shared with the VOPSD pools. Overall, the analysis of the responses shared between the two sets of N-terminus (Figure 2A) and C-terminus pools (Figure 2B) showed that the vast majority of responses (66/80) were comparable, although in a significant fraction of cases (15/80) the SFU detected with the new peptide set were higher than the ones measured with the 20mer set (≥100% increase in the total spot number).

### Detection of CD8 and CD4 T-cell responses using the VOPSD Nef peptides in HIV-infected individuals

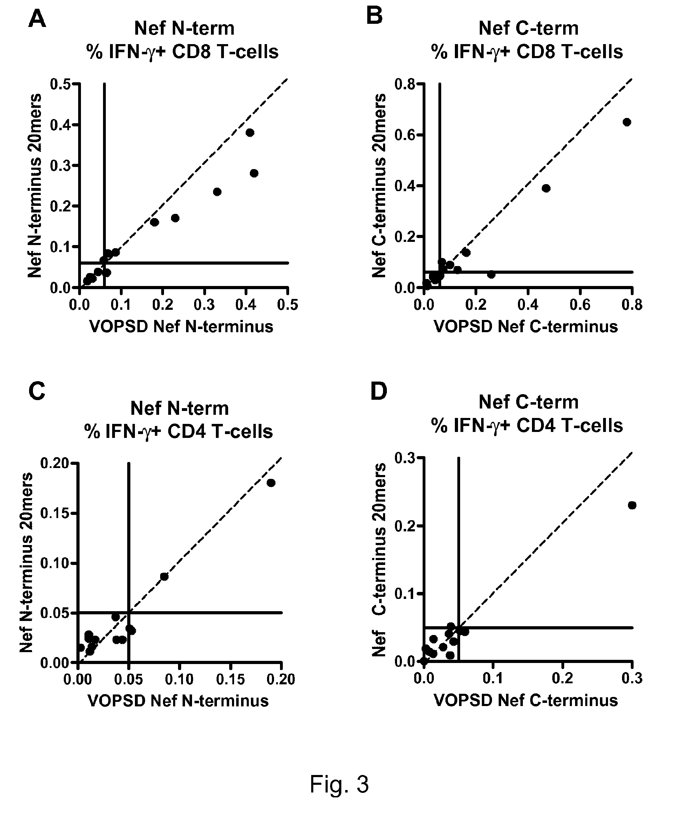

Since the Elispot approach used (IFN-γ secretion of total PBMC) do not permit to recognize the phenotype of the responding T cell, the authors extended the analysis to a smaller independent cohort of HIV-infected patients (14 individuals), using a multiparameter cytoflurimetric approach that allows combining the recognition of the T cell phenotype (CD4 or CD8) with the functional analysis (IFN-γ secretion) of the Nef specific T-cells. In this way, the authors could establish whether the higher number of individual responses measured with the new set of overlapping peptides was restricted to the CD8 compartment or interested also CD4 T-cell recognition. As shown in Figure 3, the majority of the HIV-infected patients recognized both Nef-specific peptide pools. As expected, since VOPSD has been particularly studied for the detection of MHC class I epitopes, CD8 responses measured by the new peptide set were brighter than the responses obtained with the classical 20mers pools (Figure 3A-B). Interestingly, when the CD4 T cell were considered, the new peptide set detected a higher number of responses of the pools prepared with 20mers overlapping peptides (8 and 4, respectively), a system that is considered more suited for monitoring CD4 T-cell responses.

### Higher efficiency of T-cell epitope recognition is ensured by VOPSD peptide pools

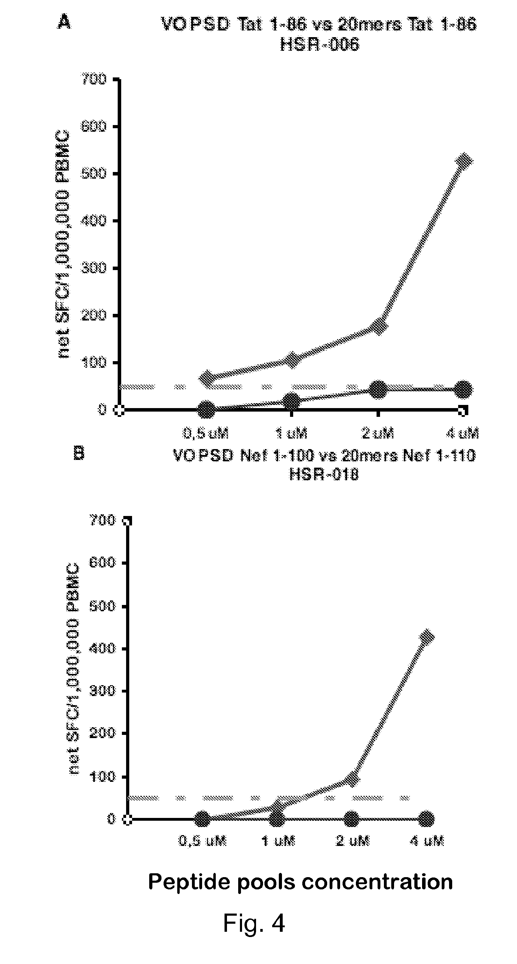

Next, the authors investigated whether the small differences in molar concentration due to the different peptide length and peptide composition of the Tat Specific (1.4µM for the VOPS pool and 1.2µM for the 20mers) and Nef-specific peptide sets (1.5 µM and 1.2 µM for the N and C-terminus VOPS pools respectively, and 1.2 µM and 1.1 µM for the homolog 20mers pools respectively) were responsible for the observed differences in HIV-encoded antigens recognition. In this regard, two patients (patient HSR-006 and 018) showing a clear cut positive recognition of the sole VOPSD Tat or Nef pools when tested at the fixed single dose concentration in IFN-γ Elispot experiment (3µg/ml for each peptide) were selected for peptide-pool titration experiments. As shown, (Figure 4A) PBMC derived from HSR-006 recognized VOPS Tat peptide pool at concentration as low as 0,5 µM. On the contrary, the homolog 20mer pool was not yet detected at concentration eight times higher. Analogously, PBMC from patients HSR-018 showed an increasing ability to recognize the VOPSD N-terminus Nef peptide pool starting from the concentration of 2µM, but failed to recognize the 20mer Nef pool at the higher concentration tested.

The authors further tested the efficacy of the VOPSD peptide design at the level of recognition of individual peptide (Figure 5) as well as of a defined minimal T-cell epitope (Figure 6A,B).

PBMC derived from patient HSR-028 that in a previous experiment were shown to recognize only peptide n°1 of the VOPSD Tat pool, were tested against increasing concentrations of VOPSD Tat peptide 1 and its 20mer homolog. Maximal recognition of VOPSD peptide 1 was reached at peptide concentrations as low as 0,5 µM whereas its homolog was recognized only at the highest concentration tested (Figure 5).

To test the efficacy of VOPSD peptides in presenting T-cell minimal epitopes, PBMC obtained from patient V4, a subject whose Nef-specific T-cell responses were previously characterized (8), were tested against all the peptides belonging either to the VOPSD or the 20mer sets of Nef-specific peptides set containing the epitope over a range of different concentrations. Subject V4 demonstrated a strong CD8 T-cell response to the B8 restricted FLKEKGGL epitope (Figure 6A). Detection of the MHC class I minimal epitope FLKEKGGL was drastically improved by the new peptide design strategy as demonstrated by the marked reduction of peptide needed for the detection of the positive response (from 2 to 0,25µM) and by the higher number of peptide specific CD8 T cell measured with the two peptides belonging to the VOPSD set (Figure 6B).

### Nef and Tat specific CD8 T-cells in LTNP produce MIP-1β but not IFN-γ and have a CD45RA+ phenotype

HIV-1 establishes a persistent chronic infection in human beings and long term survival of HIV-1 infected patients requires the administration of a special cocktail of antiretroviral drugs termed highly active antiretroviral therapy (HAART). Administration of HAART does not clear HIV-1 that, even under the strong pressure of the drugs, continues to replicate. In fact, interruption of HAART leads to a rise of the viral load that reaches pre-treatment levels in the majority of the patients. An exception to this scenario is represented by a cohort of HIV-1 infected individuals, named long-term non progressor (LTNP) that is able to naturally control viral replication and maintain a competent immune system without the help of HAART. Understanding why LTNP patients maintain an effective control of viral replication is of extreme relevance to the design of future vaccines and therapies against HIV-1.

The capacity to detect the complete repertoire of T-cells specific for a certain antigen is an important aspect for characterizing the adaptative immune response. In fact, in HIV-1 infected individuals multiple epitope recognition is associated with low steady-state viremia (14). Furthermore, functional and phenotypic heterogeneity between T-cells specific to different epitopes have been observed in several infectious diseases, such as EBV (7), HCMV (25), and

HIV-1 (12). As a consequence, the characterization of the immune responses via the analysis of few T-cell specificities may be misleading.

Given the superior ability of both Tat and Nef-specific VOPSD sets in detecting T-cell specific immune responses, the authors decided to combine the new peptide sets with a highly sophisticated polychromatic flow cytometry approach to analyze PBMC derived from HIV+ patients after stimulation with Nef and Tat peptide pools. They coupled the simultaneous detection of four functional markers, IFN-γ, IL-2, CD154 and MIP-1β with the measurement of the expression of CD45RA, a marker of T-cells maturation (Figure 7). The authors analysed the functional and differentiation phenotype of Nef and Tat specific CD8 T-cells in LTNP, late progressor (LP), chronically HIV-1 infected (CHI) individual under HAART and chronically HIV-1 infected individuals undergoing one cycle of treatment interruption. The simultaneous analysis of the immune response in these four cohorts allowed a clear discrimination between phenotypes associated with long term viral control and phenotypes that are the consequence of the exposure to the antigen.

The authors detected CD8 T-cell responses specific to Nef in the majority of the individuals. As expected, they did not find CD8 T-cells expressing CD154 and this marker was excluded from the analysis. Overall, LTNP showed higher frequencies of Nef-specific CD8 T-cells than LP and CHI, but differences resulted to be not statistically significant (data not shown). Nef-specific responses were composed of MIP-1β+ or MIP-1β+ IFN-γ+ cells while frequencies of IL-2 producing cells were generally low. Of particular interest, statistical significant differences between frequencies of CD8 T-cell responses in LTNP, LP and CHI individuals were found in CD45RA+ IFN-γ- IL-2- MIP-1β+ and CD45RA+ IFN-γ+ IL-2- MIP-1β+ CD8 T-cells. The frequency of CD45RA+ IFN-γ- IL-2- MIP-1β+ CD8 T-cells was significantly higher in LTNP than CHI with an extremely low p value (p<0.0001, Fig. 8a). A more accurate evaluation of the quality of the Nef specific response was assessed calculating the contribution of each population to the total CD8 T-cell response (Fig. 8 d, e and f). This data representation allows for the evaluation of the quality of the response independently from the magnitude. Statistical significant differences were again observed in CD45RA+ IFN-γ- IL-2-MIP-1β+ CD8 T-cells. The proportion of responding CD45RA+ IFN-γ- IL-2- MIP-1β+ CD8 T-cells was significantly higher in LTNP than CHI (p<0.0001; Fig. 8d).

A close inspection to the individual data values revealed that CD45RA+ IFN-γ- IL-2- MIP-1β+ responding CD8 T-cells were almost exclusively present in LTNP. In fact, they were observed in 7 out of 9 LTNP, 1 out of 3 LP and they were completely undetectable in all 23 CHI (Fig. 8 a and d).

Terminally differentiated CD8 T-cells, defined as CD45RA+ CCR7- that are able to express IFN-γ, have been found in LTNP (1) and in early infections with future control of HIV-1 (21). As shown in Figure 8b and e, the authors confirmed these findings. A significant higher frequency of CD45RA+ IFN-γ+ IL-2- MIP-1β+ CD8 T-cells was observed in LTNP when compared to CHI. However, the difference was not significant when the quality of the immune response was taken in consideration and CD45RA+ IFN-γ+ IL-2- MIP-1β+ CD8 T-cells were present in the three cohorts analyzed. Thus, they not constitute an exclusive marker for the LTNP status.

The frequency and proportion CD45RA- IFN-γ- IL-2- MIP-1β+ CD8 T-cell in LTNP was higher than CHI (Fig. 8c and f), nevertheless differences did not reach statistical significance. A close inspection of the percentage of the total response relative to each individual revealed a high variability between individuals belonging to the same cohort and high percentages of CD45RA- IFN-γ- IL-2- MIP-1β+ CD8 T-cell were equally observed in LTNP, LP and CHI (Fig. 8c and f).

Since IL-2 producing cells were rarely detected, the authors reanalyzed the data considering only the combination of expression of CD45RA, IFN-γ and MIP-1β in the responding CD8 T-cells. The frequency of CD45RA+ IFN-γ- MIP-1β+ CD8 T-cells was significantly higher in LTNP than in CHI (p=0.0001), Fig. 8g. In addition, the proportion of responding CD45RA+ IFN-γ- MIP-1β+ CD8 T-cells was also significantly higher in LTNP than in CHI (p=0.0001),

Fig. 8g. This analysis indicates that IL-2 expression does not represent an essential marker to define CD45RA+ IFN-γ- MIP-1β+ CD8 T-cells in LTNP.

Blood samples derived from study subjects LP02, LTNP13, LTNP14, LTNP15 and LTNP16 were drawn again nine months later and CD45RA+ IFN-γ- MIP-1β+ responding CD8 T-cells were still present; thus indicating a stability of the phenotype over the time (data not shown).

Tat specific immune responses were analyzed and as expected, Tat specific immune responses were detected only in a minority of subjects, 5/9 LTNPs, 2/3 LPs and 7/23 CHI (Fig 9a) Interestingly, CD45RA+ IFN-γ- IL-2- MIP-1β+ responding CD8 T-cells were found only in two LTNP (Fig. 9b). Overall, these data strongly support the idea that CD45RA+ IFN-γ-MIP-1β+ responding CD8 T-cells represent a positive marker of correlation for the long-term capacity of HIV-1 infected individuals to control viral replication.

### MIP-1β+ IFN-γ- IL-2- CD45RA+ CD8 T-cells are not induced by exposure to antigen load

Antigen level and persistence contribute to the determination of the function and phenotype of the responding T-cells (15). The authors' cohorts of LTNP and CHI individuals are both characterized by a prolonged exposure to HIV-1 antigens. However, the plasma viral load in LTNP was significantly higher than in CHI individuals (see Materials and Methods section). To investigate the role of the antigen level in the generation of the MIP-1β+ IFN-γ- IL-2-CD45RA+ CD8 T-cells, the authors analysed Nef and Tat specific CD8 T-cell responses after one cycle of treatment interruption in six CHI patients. Following the interruption of the treatment, the viral loads became detectable in all the patients within day 5 and 21. Therapy was resumed with viral loads higher than 100,000 RNA copies/ml. In general, Nef specific CD8 T-cell response followed the peak of the viral load. A significant increase in the frequency of CD45RA+ IFN-γ+ IL-2- MIP-1β+ (p=0.0313) and CD45RA- IFN-γ+ IL-2-MIP-1β+ CD8 T-cells (p=0.0313) was observed, while CD45RA+ IFN-γ- IL-2- MIP-1β+ CD8 T-cells remained undetectable (Fig. 10). Interestingly, the quality of the CD8 immune response remained unchanged despite the significant increase observed in the magnitude of the response. Immune response to Tat resulted to be almost undetectable before and after the interruption of the therapy (data not shown). These data demonstrate that MIP-1β+ IFN-γ- IL-2- CD45RA+ CD8 T-cells are not directly induced by the exposure to the antigen.

### VOPSD peptides enhance detection of CD45RA+ IFN-γ- IL-2- MIP-1β+ CD8 T-cells

The authors have shown that VOPSD peptides are superior in detecting CD8 and CD4 responses using an IFN-γ-based ELISPOT and an IFN-γ-based ICS. The combination of the use of the VOPSD peptides and of the immunocytometric assay lead to the identification of the CD45RA+ IFN-γ- IL-2- MIP-1β+ CD8 T-cell population almost exclusively present in LTNP. The question arises whether the use of the VOPSD peptides increases the capacity to detect not only IFN-γ+ CD8 T-cells but also CD45RA+ IFN-γ- IL-2- MIP-1β+ CD8 T-cells. To this aim, the authors compared Nef responses after stimulation with the VOPSD, a pool of Nef derived optimal CD8 T-cell epitopes (ref. 8) and the homolog 20mer set of Nef peptides in a subject with a strong Nef specific response (subject V4). The optimal pool was a set of peptides representing 16 optimally defined Nef CD8 epitopes (ref. 8; WPTVRERM : SEQ ID No. 135; ALTSSNTAA : SEQ ID No. 136; FPVTPQVPLR : SEQ ID No. 137; FPVRPQVPL : SEQ ID No. 138; RPQVPLRPMTY : SEQ ID No. 139; QVPLRPMTYK : SEQ ID No. 140; VPLRPMTY : SEQ ID No. 141; RPMTYKAAL : SEQ ID No. 142; AVDLSHFLK : SEQ ID No. 143; FLKEKGGL : SEQ ID No. 144; RRQDILDLWI : SEQ ID No. 145; TPGPGVRYPL : SEQ ID No. 146; YPLTFGWCY : SEQ ID No. 147; PLTFGWCFKL : SEQ ID No. 148; LTFGWCFKL : SEQ ID No. 149; VLEWRFDSRL : SEQ ID No. 150).

As shown in Figure 11, higher frequencies of responses were detected using the VOPDS peptides. The authors detected 0.83% CD45RA+ IFN-γ- IL-2- MIP-1β+ CD8 T-cells using the VOPSD peptides, 0.55% CD45RA+ IFN-γ- IL-2- MIP-1β+ CD8 T-cells using the optimal pool and 0.38% CD45RA+ IFN-γ- IL-2- MIP-1β+ CD8 T-cells using the 20mer set of peptides.

These data indicate that VOPSD peptides increase the probability to detect CD45RA+ IFN-γ-IL-2- MIP-1β+ CD8 T-cells.

### REFERENCES

1. Addo, M. M., et al., 2007. PLoS ONE 2:e321.
2. Addo, M. M., et al., 2003. J Virol 77:2081-92.
3. Baba, T. W., et al., 2000. Nat Med 6:200-6.
4. Barouch, D. H., et al., 2000. Science 290:486-92.
5. Beattie, T., et al., 2004. Aids 18:1595-8.
6. Betts, M. R., et al., 2001. J Virol 75:11983-91.
7. Catalina, M. D., et al., 2002. J Immunol 168:4184-91.
8. Cosma, A., et al., 2003. Vaccine 22:21-9.
9. De Groot, A. S., et al., 2003. Vaccine 21:4486-504.
10. Draenert, R., et al., 2003. J Immunol Methods 275:19-29.
11. Draenert, R., C. et al., 2004. Aids 18:871-6.
12. Fujiwara, M., et al., 2005. J Virol 79:12536-43.
13. Gaschen, B., et al., 2002. Science 296:2354-60.
14. Geldmacher, C., et al., 2007. J Virol 81:2440-8.
15. Harari, A., et al., 2006. Immunol Rev 211:236-54.
16. Koup, R. A., et al., 1994. J Virol 68:4650-5.
17. Malhotra, U., et al., 2007. J Virol 81:5225-37.
18. Mazzoli, S., et al., 1997. Nat Med 3:1250-7.
19. McMichael, A. J., and R. E. Phillips. 1997. Annu Rev Immunol 15:271-96.
20. Musey, L., et al., 1997. N Engl J Med 337:1267-74.
21. Northfield, J. W., et al., 2007. J Virol 81:5759-65.
22**.** Ogg, G. S., et al., 1998. Science 279:2103-6.
23. Schmittel, A., et al., 1997. J Immunol Methods 210:167-74.
24**.** Schmitz, J. E., et al., 1999. Science 283:857-60.
25. Wills, M. R., et al., 2002. J Immunol 168:5455-64.
26. Vardas E. et al., 2005. Microbes and Infec. 7:1436-1444.

## Claims

1. A method for designing a set of overlapping peptides of an antigen comprising the steps of:
a) aligning the amino acid sequence of a variant of the antigen with one amino acid consensus sequence of the same antigen;
b) mapping on said aligned amino acid sequences known immunodominant regions and optimal epitope regions;
c) individuating a first sub-set of peptides consisting of conserved sequence regions between the amino acid sequence of the variant and the amino acid consensus sequence of the variable antigen, said peptides being longer than 10 amino acids ;
d) completing the overlapping peptide design by individuating a second sub-set of peptides, each peptide containing a protein fragment of the amino acid sequence of the variant that differs for one or more amino acid residues from the amino acid consensus sequence of the variable antigen, and optionally one or more immunodominant region and/or a optimal epitope region or portions thereof at its N or C terminus,
wherein each of said peptides of said second sub-set of peptides is between 10 and 19 amino acids long and does not have as the last C-terminal amino acid any of the following amino acids: Asn, Asp, Gln, Glu, Gly, His, Ser;
and wherein each of the set of overlapping peptides has a gap not longer than 8 amino acids with its closest overlapping peptide.

2. The method of claim 1 wherein the variable antigen is an HIV antigen.

3. The method of claim 2 wherein the HIV antigen is an HIV-1 antigen.

4. The method of claim 3 wherein the HIV antigen is an HIV-1 Tat antigen or a Nef antigen.

5. A set of overlapping peptides obtainable according to the method of claim 1 to 4.

6. The set of overlapping peptides according to claim 5 being of the variable Tat antigen of HIV-1.

7. The set of overlapping peptides according to claim 6 consisting of peptides belonging to any of the following groups:
a) peptides of SEQ ID No. 1 to SEQ ID No. 11;
b) peptides of SEQ ID No. 42 to SEQ ID No. 51 and SEQ ID No. 7.

8. The set of overlapping peptides according to claim 5 being of the variable Nef antigen of HIV-1.

9. The set of overlapping peptides according to claim 8 consisting of peptides belonging to any of the following groups:
a) peptides of SEQ ID No. 12 to SEQ ID No. 26;
b) peptides of SEQ ID No. 27 to SEQ ID No. 41;
c) peptides of SEQ ID No. 12 to SEQ ID No. 41;
d) peptides of SEQ ID No. 52 to SEQ ID No.63 and SEQ ID No. 20, SEQ ID No. 23 and SEQ ID No. 26;
e) peptides of SEQ ID No. 64 to SEQ ID No. 76 and SEQ ID No. 30, SEQ ID No. 31 and SEQ ID No.32;
f) peptides of SEQ ID No. 52 to SEQ ID No. 76 and SEQ ID No. 20, SEQ ID No. 23, SEQ ID No. 26, SEQ ID No. 30, SEQ ID No. 31 and SEQ ID No.32;
g) peptides of SEQ ID No. 77 to SEQ ID No. 86.

10. The set of overlapping peptides according to claims 5 to 9 for use as a medicament, a vaccine or a vaccine adjuvant.

11. Use of the set of overlapping peptides according to claims 5 to 9 for monitoring CD4 and/or CD8 T cell responses in HIV patients ex vivo.

12. Use of the set of overlapping peptides according to claim 5 to 9 for identifying and/or selecting and/or monitoring a HIV-1 negative population for anti HIV-1 vaccine development.

13. Use of the set of overlapping peptides according to claims 5 to 9 for the detection, in a biological sample, of a population of T cells belonging to any of the following groups:
a) CD4 T cells;
b) CD8 T cells;
c) CD45RA⁺IFNγ⁻ MIP1β⁺ CD8 T cells.

14. An isolated population of CD45RA⁺IFNγ MIP1β⁺ CD8 T cells.

15. Use of a CD45RA⁺IFNγ MIP1β⁺ CD8 T cell population as immune therapy.

16. A kit to detect a CD45RA⁺ IFNγ⁻ MIP1β⁺ CD8 T cell population in a biological sample comprising a set of peptides belonging to any of the following groups:
a) a set of overlapping peptides according to claims 5 to 9;
b) standard pools of overlapping peptides;
c) pools of optimal CD8 T-cell epitopes.

17. A method for detecting a CD45RA⁺ IFNγ⁻ MIP1β⁺ CD8 T cell population in a biological sample comprising incubating said sample with a set of peptides belonging to any of the following groups:
a) a set of overlapping peptides according to claims 5 to 9;
b) standard pools of overlapping peptides;
c) pools of optimal CD8 T-cell epitopes.

18. A method for the diagnostic of a long-term non-progressive (LTNP) status, or for monitoring the anti-HIV-specific protective CD8 T cell response, in a biological sample from a HIV-1 infected subject, comprising the steps of:
a) detecting the CD45RA⁺IFNγ⁻ MIP1β⁺ CD8 T cell population according to claim 17, and
b) measuring the % thereof with respect to the total CD8 T cells or to the total responding CD8 T cells.

19. A method for measuring the efficacy of an HIV derived antigen vaccine in a biological sample from a vaccinated HIV-1 infected subject, comprising the steps of:
a) detecting the CD45RA⁺IFNγ⁻ MIP1β⁺ CD8 T cell population according to claim 17, and
b) measuring the % thereof with respect to the total CD8 T cells or to the total responding CD8 T cells.


SEQUENCE LISTING

<110>  Fondazione Centro San Raffaele Del Monte Tabor
       Helmholtz Zentrum Munchen Deutsches Forschungszentrum fur
       Gesundheit und Umwelt (GmbH)
       MALNATI, Mauro Severo
       LUSSO, Paolo
       POLI, Guido
       COSMA, Antonio
       ERFLE, Volker

<120>  Method to design overlapping peptides, overlapping peptides and
       uses thereof

<130>  BE 101445

<160>  150

<170>  PatentIn version 3.3

<210>  1
<211>  11
<212>  PRT
<213>  Artificial

<220>
<223>  synthetic peptide

<400>  1

Met Glu Pro Val Asp Pro Arg Leu Glu Pro Trp
1               5                   10


<210>  2
<211>  19
<212>  PRT
<213>  Artificial

<220>
<223>  synthetic peptide

<400>  2

Leu Glu Pro Trp Lys His Pro Gly Ser Gln Pro Lys Thr Ala Cys Thr
1               5                   10                  15


Asn Cys Tyr



<210>  3
<211>  18
<212>  PRT
<213>  Artificial

<220>
<223>  synthetic peptide

<400>  3

Gly Ser Gln Pro Lys Thr Ala Cys Thr Asn Cys Tyr Cys Lys Lys Cys
1               5                   10                  15


Cys Phe

<210>  4
<211>  17
<212>  PRT
<213>  Artificial

<220>
<223>  synthetic peptide

<400>  4

Ala Cys Thr Asn Cys Tyr Cys Lys Lys Cys Cys Phe His Cys Gln Val
1               5                   10                  15


Cys



<210>  5
<211>  18
<212>  PRT
<213>  Artificial

<220>
<223>  synthetic peptide

<400>  5

Lys Lys Cys Cys Phe His Cys Gln Val Cys Phe Ile Thr Lys Ala Leu
1               5                   10                  15


Gly Ile



<210>  6
<211>  15
<212>  PRT
<213>  Artificial

<220>
<223>  synthetic peptide

<400>  6

Gln Val Cys Phe Ile Thr Lys Ala Leu Gly Ile Ser Tyr Gly Arg
1               5                   10                  15


<210>  7
<211>  14
<212>  PRT
<213>  Artificial

<220>
<223>  synthetic peptide

<400>  7

Leu Gly Ile Ser Tyr Gly Arg Lys Lys Arg Arg Gln Arg Arg
1               5                   10


<210>  8
<211>  18
<212>  PRT
<213>  Artificial

<220>
<223>  synthetic peptide

<400>  8

Lys Lys Arg Arg Gln Arg Arg Arg Pro Pro Gln Gly Ser Gln Thr His
1               5                   10                  15

Gln Val

<210>  9
<211>  18
<212>  PRT
<213>  Artificial

<220>
<223>  synthetic peptide

<400>  9

Arg Pro Pro Gln Gly Ser Gln Thr His Gln Val Ser Leu Ser Lys Gln
1               5                   10                  15

Pro Thr

<210>  10
<211>  18
<212>  PRT
<213>  Artificial

<220>
<223>  synthetic peptide

<400>  10

His Gln Val Ser Leu Ser Lys Gln Pro Thr Ser Gln Ser Arg Gly Asp
1               5                   10                  15

Pro Thr

<210>  11
<211>  15
<212>  PRT
<213>  Artificial

<220>
<223>  synthetic peptide

<400>  11

Lys Gln Pro Thr Ser Gln Ser Arg Gly Asp Pro Thr Gly Pro Lys
1               5                   10                  15

<210>  12
<211>  17
<212>  PRT
<213>  Artificial

<220>
<223>  synthetic peptide

<400>  12

Met Gly Gly Lys Trp Ser Lys Ser Ser Val Val Gly Trp Pro Thr Val
1               5                   10                  15

Arg

<210>  13
<211>  19
<212>  PRT
<213>  Artificial

<220>
<223>  synthetic peptide

<400>  13

Ser Val Val Gly Trp Pro Thr Val Arg Glu Arg Met Arg Arg Ala Glu
1               5                   10                  15

Pro Ala Ala

<210>  14
<211>  18
<212>  PRT
<213>  Artificial

<220>
<223>  synthetic peptide

<400>  14

Val Arg Glu Arg Met Arg Arg Ala Glu Pro Ala Ala Asp Gly Val Gly
1               5                   10                  15

Ala Ala

<210>  15
<211>  17
<212>  PRT
<213>  Artificial

<220>
<223>  synthetic peptide

<400>  15

Ala Glu Pro Ala Ala Asp Gly Val Gly Ala Ala Ser Arg Asp Leu Glu
1               5                   10                  15

Lys

<210>  16
<211>  16

<212>  PRT
<213>  Artificial

<220>
<223>  synthetic peptide

<400>  16

Gly Val Gly Ala Ala Ser Arg Asp Leu Glu Lys His Gly Ala Ile Thr
1               5                   10                  15


<210>  17
<211>  18
<212>  PRT
<213>  Artificial

<220>
<223>  synthetic peptide

<400>  17

Asp Leu Glu Lys His Gly Ala Ile Thr Ser Ser Asn Thr Ala Ala Thr
1               5                   10                  15


Asn Ala



<210>  18
<211>  16
<212>  PRT
<213>  Artificial

<220>
<223>  synthetic peptide

<400>  18

Ile Thr Ser Ser Asn Thr Ala Ala Thr Asn Ala Ala Cys Ala Trp Leu
1               5                   10                  15


<210>  19
<211>  17
<212>  PRT
<213>  Artificial

<220>
<223>  synthetic peptide

<400>  19

Ala Thr Asn Ala Ala Cys Ala Trp Leu Glu Ala Gln Glu Glu Glu Glu
1               5                   10                  15


Val



<210>  20
<211>  11
<212>  PRT
<213>  Artificial

<220>
<223>  synthetic peptide

<400>  20

Cys Ala Trp Leu Glu Ala Gln Glu Glu Glu Glu
1               5                   10


<210>  21
<211>  17
<212>  PRT
<213>  Artificial

<220>
<223>  synthetic peptide

<400>  21

Gln Glu Glu Glu Glu Val Gly Phe Pro Val Thr Pro Gln Val Pro Leu
1               5                   10                  15


Arg



<210>  22
<211>  17
<212>  PRT
<213>  Artificial

<220>
<223>  synthetic peptide

<400>  22

Val Gly Phe Pro Val Thr Pro Gln Val Pro Leu Arg Pro Met Thr Tyr
1               5                   10                  15


Lys



<210>  23
<211>  10
<212>  PRT
<213>  Artificial

<220>
<223>  synthetic peptide

<400>  23

Pro Gln Val Pro Leu Arg Pro Met Thr Tyr
1               5                   10


<210>  24
<211>  16
<212>  PRT
<213>  Artificial

<220>
<223>  synthetic peptide

<400>  24

Arg Pro Met Thr Tyr Lys Ala Ala Val Asp Leu Ser His Phe Leu Lys
1               5                   10                  15

<210>  25
<211>  17
<212>  PRT
<213>  Artificial

<220>
<223>  synthetic peptide

<400>  25

Ala Val Asp Leu Ser His Phe Leu Lys Glu Lys Gly Gly Leu Glu Gly
1               5                   10                  15

Leu

<210>  26
<211>  12
<212>  PRT
<213>  Artificial

<220>
<223>  synthetic peptide

<400>  26

Phe Leu Lys Glu Lys Gly Gly Leu Glu Gly Leu Ile
1               5                   10

<210>  27
<211>  19
<212>  PRT
<213>  Artificial

<220>
<223>  synthetic peptide

<400>  27

Leu Glu Gly Leu Ile His Ser Gln Arg Arg Gln Asp Ile Leu Asp Leu
1               5                   10                  15

Trp Ile Tyr

<210>  28
<211>  17
<212>  PRT
<213>  Artificial

<220>
<223>  synthetic peptide

<400>  28

Gln Arg Arg Gln Asp Ile Leu Asp Leu Trp Ile Tyr His Thr Gln Gly
1               5                   10                  15

Tyr

<210>  29
<211>  18
<212>  PRT
<213>  Artificial

<220>
<223>  synthetic peptide

<400>  29

Leu Asp Leu Trp Ile Tyr His Thr Gln Gly Tyr Phe Pro Asp Trp Gln
1               5                   10                  15

Asn Tyr

<210>  30
<211>  14
<212>  PRT
<213>  Artificial

<220>
<223>  synthetic peptide

<400>  30

Tyr His Thr Gln Gly Tyr Phe Pro Asp Trp Gln Asn Tyr Thr
1               5                   10

<210>  31
<211>  16
<212>  PRT
<213>  Artificial

<220>
<223>  synthetic peptide

<400>  31

Tyr Phe Pro Asp Trp Gln Asn Tyr Thr Pro Gly Pro Gly Val Arg Tyr
1               5                   10                  15

<210>  32
<211>  17
<212>  PRT
<213>  Artificial

<220>
<223>  synthetic peptide

<400>  32

Gln Asn Tyr Thr Pro Gly Pro Gly Val Arg Tyr Pro Leu Thr Phe Gly
1               5                   10                  15

Trp

<210>  33
<211>  16
<212>  PRT
<213>  Artificial

<220>
<223>  synthetic peptide

<400>  33

Gly Pro Gly Val Arg Tyr Pro Leu Thr Phe Gly Trp Cys Tyr Lys Leu
1               5                   10                  15


<210>  34
<211>  19
<212>  PRT
<213>  Artificial

<220>
<223>  synthetic peptide

<400>  34

Thr Phe Gly Trp Cys Tyr Lys Leu Val Pro Val Glu Pro Asp Lys Val
1               5                   10                  15


Glu Glu Ala



<210>  35
<211>  19
<212>  PRT
<213>  Artificial

<220>
<223>  synthetic peptide

<400>  35

Val Pro Val Glu Pro Asp Lys Val Glu Glu Ala Asn Lys Gly Glu Asn
1               5                   10                  15


Thr Ser Leu



<210>  36
<211>  17
<212>  PRT
<213>  Artificial

<220>
<223>  synthetic peptide

<400>  36

Lys Val Glu Glu Ala Asn Lys Gly Glu Asn Thr Ser Leu Leu His Pro
1               5                   10                  15


Val



<210>  37
<211>  19
<212>  PRT
<213>  Artificial

<220>
<223>  synthetic peptide

<400>  37

Glu Asn Thr Ser Leu Leu His Pro Val Ser Leu His Gly Met Asp Asp
1               5                   10                  15

Pro Glu Arg

<210>  38
<211>  18
<212>  PRT
<213>  Artificial

<220>
<223>  synthetic peptide

<400>  38

Pro Val Ser Leu His Gly Met Asp Asp Pro Glu Arg Glu Val Leu Glu
1               5                   10                  15

Trp Arg

<210>  39
<211>  18
<212>  PRT
<213>  Artificial

<220>
<223>  synthetic peptide

<400>  39

Asp Asp Pro Glu Arg Glu Val Leu Glu Trp Arg Phe Asp Ser Arg Leu
1               5                   10                  15

Ala Phe

<210>  40
<211>  19
<212>  PRT
<213>  Artificial

<220>
<223>  synthetic peptide

<400>  40

Val Leu Glu Trp Arg Phe Asp Ser Arg Leu Ala Phe His His Val Ala
1               5                   10                  15

Arg Glu Leu

<210>  41
<211>  18

<212>  PRT
<213>  Artificial

<220>
<223>  synthetic peptide

<400>  41

Asp Ser Arg Leu Ala Phe His His Val Ala Arg Glu Leu His Pro Glu
1               5                   10                  15

Tyr Phe

<210>  42
<211>  11
<212>  PRT
<213>  Artificial

<220>
<223>  synthetic peptide

<400>  42

Met Glu Pro Val Asp Pro Asn Leu Glu Pro Trp
1               5                   10

<210>  43
<211>  19
<212>  PRT
<213>  Artificial

<220>
<223>  synthetic peptide

<400>  43

Leu Glu Pro Trp Asn His Pro Gly Ser Gln Pro Lys Thr Ala Cys Asn
1               5                   10                  15

Thr Cys Tyr

<210>  44
<211>  18
<212>  PRT
<213>  Artificial

<220>
<223>  synthetic peptide

<400>  44

Gly Ser Gln Pro Lys Thr Ala Cys Asn Thr Cys Tyr Cys Lys Lys Cys
1               5                   10                  15

Ser Tyr

<210>  45
<211>  18
<212>  PRT

<213>  Artificial

<220>
<223>  synthetic peptide

<400>  45

Ala Cys Asn Thr Cys Tyr Cys Lys Lys Cys Ser Tyr His Cys Leu Val
1               5                   10                  15

Cys Phe

<210>  46
<211>  18
<212>  PRT
<213>  Artificial

<220>
<223>  synthetic peptide

<400>  46

Lys Lys Cys Ser Tyr His Cys Leu Val Cys Phe Gln Thr Lys Gly Leu
1               5                   10                  15

Gly Ile

<210>  47
<211>  15
<212>  PRT
<213>  Artificial

<220>
<223>  synthetic peptide

<400>  47

Leu Val Cys Phe Ile Thr Lys Gly Leu Gly Ile Ser Tyr Gly Arg
1               5                   10                  15

<210>  48
<211>  18
<212>  PRT
<213>  Artificial

<220>
<223>  synthetic peptide

<400>  48

Lys Lys Arg Arg Gln Arg Arg Ser Ala Pro Pro Ser Ser Glu Asp His
1               5                   10                  15

Gln Asn

<210>  49
<211>  18
<212>  PRT
<213>  Artificial

<220>
<223>  synthetic peptide

<400>  49

Ser Ala Pro Pro Ser Ser Glu Asp His Gln Asn Pro Ile Ser Lys Gln
1               5                   10                  15

Pro Leu

<210>  50
<211>  18
<212>  PRT
<213>  Artificial

<220>
<223>  synthetic peptide

<400>  50

His Gln Asn Pro Ile Ser Lys Gln Pro Leu Pro Arg Thr Leu Gly Asp
1               5                   10                  15

Pro Thr

<210>  51
<211>  15
<212>  PRT
<213>  Artificial

<220>
<223>  synthetic peptide

<400>  51

Lys Gln Pro Leu Pro Arg Thr Leu Gly Asp Pro Thr Gly Ser Glu
1               5                   10                  15

<210>  52
<211>  17
<212>  PRT
<213>  Artificial

<220>
<223>  synthetic peptide

<400>  52

Met Gly Gly Lys Trp Ser Lys Cys Ser Ile Val Gly Trp Pro Ala Val
1               5                   10                  15

Arg

<210>  53
<211>  19
<212>  PRT
<213>  Artificial

<220>
<223>  synthetic peptide

<400>  53

Ser Ile Val Gly Trp Pro Ala Val Arg Glu Arg Met Arg Arg Thr Glu
1               5                   10                  15

Pro Ala Ala

<210>  54
<211>  18
<212>  PRT
<213>  Artificial

<220>
<223>  synthetic peptide

<400>  54

Val Arg Glu Arg Met Arg Arg Thr Glu Pro Ala Ala Glu Gly Val Gly
1               5                   10                  15

Ala Ala

<210>  55
<211>  17
<212>  PRT
<213>  Artificial

<220>
<223>  synthetic peptide

<400>  55

Thr Glu Pro Ala Ala Glu Gly Val Gly Ala Ala Ser Gln Asp Leu Asp
1               5                   10                  15

Lys

<210>  56
<211>  16
<212>  PRT
<213>  Artificial

<220>
<223>  synthetic peptide

<400>  56

Gly Val Gly Ala Ala Ser Gln Asp Leu Asp Lys His Gly Ala Leu Thr
1               5                   10                  15

<210>  57
<211>  18
<212>  PRT
<213>  Artificial

<220>

<223>  synthetic peptide

<400>  57

Asp Leu Asp Lys His Gly Ala Leu Thr Ser Ser Asn Thr Ala Ala Asn
1               5                   10                  15

Asn Ala

<210>  58
<211>  16
<212>  PRT
<213>  Artificial

<220>
<223>  synthetic peptide

<400>  58

Leu Thr Ser Ser Asn Thr Ala Ala Asn Asn Ala Asp Cys Ala Trp Leu
1               5                   10                  15

<210>  59
<211>  18
<212>  PRT
<213>  Artificial

<220>
<223>  synthetic peptide

<400>  59

Ala Asn Asn Ala Asp Cys Ala Trp Leu Glu Ala Gln Glu Glu Glu Glu
1               5                   10                  15

Glu Val

<210>  60
<211>  18
<212>  PRT
<213>  Artificial

<220>
<223>  synthetic peptide

<400>  60

Gln Glu Glu Glu Glu Glu Val Gly Phe Pro Val Arg Pro Gln Val Pro
1               5                   10                  15

Leu Arg

<210>  61
<211>  17
<212>  PRT
<213>  Artificial

<220>
<223>  synthetic peptide

<400>  61

Val Gly Phe Pro Val Arg Pro Gln Val Pro Leu Arg Pro Met Thr Tyr
1               5                   10                  15

Lys

<210>  62
<211>  16
<212>  PRT
<213>  Artificial

<220>
<223>  synthetic peptide

<400>  62

Arg Pro Met Thr Tyr Lys Ala Ala Phe Asp Leu Ser Phe Phe Leu Lys
1               5                   10                  15

<210>  63
<211>  17
<212>  PRT
<213>  Artificial

<220>
<223>  synthetic peptide

<400>  63

Ala Phe Asp Leu Ser Phe Phe Leu Lys Glu Lys Gly Gly Leu Glu Gly
1               5                   10                  15

Leu

<210>  64
<211>  19
<212>  PRT
<213>  Artificial

<220>
<223>  synthetic peptide

<400>  64

Leu Glu Gly Leu Ile Tyr Ser Lys Lys Arg Gln Glu Ile Leu Asp Leu
1               5                   10                  15

Trp Val Tyr

<210>  65
<211>  17
<212>  PRT
<213>  Artificial

<220>
<223>  synthetic peptide

<400>  65

Lys Lys Arg Gln Glu Ile Leu Asp Leu Trp Val Tyr His Thr Gln Gly
1               5                   10                  15

Tyr

<210>  66
<211>  18
<212>  PRT
<213>  Artificial

<220>
<223>  synthetic peptide

<400>  66

Leu Asp Leu Trp Val Tyr His Thr Gln Gly Tyr Phe Pro Asp Trp Gln
1               5                   10                  15

Asn Tyr

<210>  67
<211>  16
<212>  PRT
<213>  Artificial

<220>
<223>  synthetic peptide

<400>  67

Gly Pro Gly Val Arg Tyr Pro Leu Thr Phe Gly Trp Cys Phe Lys Leu
1               5                   10                  15

<210>  68
<211>  18
<212>  PRT
<213>  Artificial

<220>
<223>  synthetic peptide

<400>  68

Thr Phe Gly Trp Cys Phe Lys Leu Val Pro Val Asp Pro Arg Glu Val
1               5                   10                  15

Glu Glu

<210>  69
<211>  19
<212>  PRT
<213>  Artificial

<220>
<223>  synthetic peptide

<400>  69

Val Pro Val Asp Pro Arg Glu Val Glu Glu Ala Asn Glu Gly Glu Asn
1               5                   10                  15

Asn Cys Leu

<210>  70
<211>  17
<212>  PRT
<213>  Artificial

<220>
<223>  synthetic peptide

<400>  70

Glu Val Glu Glu Ala Asn Glu Gly Glu Asn Asn Cys Leu Leu His Pro
1               5                   10                  15

Met

<210>  71
<211>  19
<212>  PRT
<213>  Artificial

<220>
<223>  synthetic peptide

<400>  71

Glu Asn Asn Cys Leu Leu His Pro Met Ser Gln His Gly Met Glu Asp
1               5                   10                  15

Glu His Arg

<210>  72
<211>  18
<212>  PRT
<213>  Artificial

<220>
<223>  synthetic peptide

<400>  72

Pro Met Ser Gln His Gly Met Glu Asp Glu His Arg Glu Val Leu Lys
1               5                   10                  15

Trp Lys

<210>  73
<211>  18
<212>  PRT
<213>  Artificial

<220>

<223>  synthetic peptide

<400>  73

Glu Asp Glu His Arg Glu Val Leu Lys Trp Lys Phe Asp Ser His Leu
1               5                   10                  15

Ala Arg

<210>  74
<211>  19
<212>  PRT
<213>  Artificial

<220>
<223>  synthetic peptide

<400>  74

Val Leu Lys Trp Lys Phe Asp Ser His Leu Ala Arg Arg His Met Ala
1               5                   10                  15

Arg Glu Leu

<210>  75
<211>  18
<212>  PRT
<213>  Artificial

<220>
<223>  synthetic peptide

<400>  75

Asp Ser His Leu Ala Arg Arg His Met Ala Arg Glu Leu His Pro Glu
1               5                   10                  15

Tyr Tyr

<210>  76
<211>  15
<212>  PRT
<213>  Artificial

<220>
<223>  synthetic peptide

<400>  76

Arg His Met Ala Arg Glu Leu His Pro Glu Tyr Tyr Lys Asp Cys
1               5                   10                  15

<210>  77
<211>  14
<212>  PRT
<213>  Artificial

<220>
<223>  synthetic peptide

<400>  77

Tyr His Thr Gln Gly Phe Phe Pro Asp Trp Gln Asn Tyr Thr
1               5                   10


<210>  78
<211>  16
<212>  PRT
<213>  Artificial

<220>
<223>  synthetic peptide

<400>  78

Phe Phe Pro Asp Trp Gln Asn Tyr Thr Pro Gly Pro Gly Val Arg Tyr
1               5                   10                  15


<210>  79
<211>  18
<212>  PRT
<213>  Artificial

<220>
<223>  synthetic peptide

<400>  79

Asp Leu Asp Lys His Gly Ala Leu Thr Ser Ser Asn Thr Ala His Asn
1               5                   10                  15


Asn Ala



<210>  80
<211>  16
<212>  PRT
<213>  Artificial

<220>
<223>  synthetic peptide

<400>  80

Leu Thr Ser Ser Asn Thr Ala His Asn Asn Ala Asp Cys Ala Trp Leu
1               5                   10                  15


<210>  81
<211>  18
<212>  PRT
<213>  Artificial

<220>
<223>  synthetic peptide

<400>  81

His Asn Asn Ala Asp Cys Ala Trp Leu Glu Ala Gln Glu Glu Glu Glu
1               5                   10                  15


Glu Val

<210>  82
<211>  18
<212>  PRT
<213>  Artificial

<220>
<223>  synthetic peptide

<400>  82

Leu Asp Leu Trp Val Tyr His Thr Gln Gly Phe Phe Pro Asp Trp Gln
1               5                   10                  15

Asn Tyr

<210>  83
<211>  18
<212>  PRT
<213>  Artificial

<220>
<223>  synthetic peptide

<400>  83

Pro Met Ser Gln His Gly Met Glu Asp Glu Asp Arg Glu Val Leu Lys
1               5                   10                  15

Trp Lys

<210>  84
<211>  18
<212>  PRT
<213>  Artificial

<220>
<223>  synthetic peptide

<400>  84

Glu Asp Glu Asp Arg Glu Val Leu Lys Trp Lys Phe Asp Ser Ser Leu
1               5                   10                  15

Ala Arg

<210>  85
<211>  19
<212>  PRT
<213>  Artificial

<220>
<223>  synthetic peptide

<400>  85

Val Leu Lys Trp Lys Phe Asp Ser Ser Leu Ala Arg Arg His Met Ala
1               5                   10                  15

Arg Glu Leu

<210> 86
<211> 18
<212> PRT
<213> Artificial

<220>
<223> synthetic peptide

<400> 86

Asp Ser Ser Leu Ala Arg Arg His Met Ala Arg Glu Leu His Pro Glu
1               5                   10                  15

Tyr Tyr

<210> 87
<211> 15
<212> PRT
<213> Artificial

<220>
<223> synthetic peptide

<400> 87

Met Glu Pro Val Asp Pro Arg Leu Glu Pro Trp Lys His Pro Gly
1               5                   10                  15

<210> 88
<211> 15
<212> PRT
<213> Artificial

<220>
<223> synthetic peptide

<400> 88

Pro Arg Leu Glu Pro Trp Lys His Pro Gly Ser Gln Pro Lys Thr
1               5                   10                  15

<210> 89
<211> 15
<212> PRT
<213> Artificial

<220>
<223> synthetic peptide

<400> 89

Trp Lys His Pro Gly Ser Gln Pro Lys Thr Ala Cys Thr Asn Cys
1               5                   10                  15

<210> 90
<211> 15
<212> PRT
<213> Artificial

<220>
<223>  synthetic peptide

<400>  90

Ser Gln Pro Lys Thr Ala Cys Thr Asn Cys Tyr Cys Lys Lys Cys
1               5                   10                  15


<210>  91
<211>  15
<212>  PRT
<213>  Artificial

<220>
<223>  synthetic peptide

<400>  91

Ala Cys Thr Asn Cys Tyr Cys Lys Lys Cys Cys Phe His Cys Gln
1               5                   10                  15


<210>  92
<211>  15
<212>  PRT
<213>  Artificial

<220>
<223>  synthetic peptide

<400>  92

Tyr Cys Lys Lys Cys Cys Phe His Cys Gln Val Cys Phe Ile Thr
1               5                   10                  15


<210>  93
<211>  15
<212>  PRT
<213>  Artificial

<220>
<223>  synthetic peptide

<400>  93

Cys Phe His Cys Gln Val Cys Phe Ile Thr Lys Ala Leu Gly Ile
1               5                   10                  15


<210>  94
<211>  15
<212>  PRT
<213>  Artificial

<220>
<223>  synthetic peptide

<400>  94

Val Cys Phe Ile Thr Lys Ala Leu Gly Ile Ser Tyr Gly Arg Lys
1               5                   10                  15


<210>  95
<211>  15
<212>  PRT

<213>  Artificial

<220>
<223>  synthetic peptide

<400>  95

Lys Ala Leu Gly Ile Ser Tyr Gly Arg Lys Lys Arg Arg Gln Arg
1               5                   10                  15


<210>  96
<211>  15
<212>  PRT
<213>  Artificial

<220>
<223>  synthetic peptide

<400>  96

Ser Tyr Gly Arg Lys Lys Arg Arg Gln Arg Arg Arg Pro Pro Gln
1               5                   10                  15


<210>  97
<211>  15
<212>  PRT
<213>  Artificial

<220>
<223>  synthetic peptide

<400>  97

Lys Arg Arg Gln Arg Arg Arg Pro Pro Gln Gly Ser Gln Thr His
1               5                   10                  15


<210>  98
<211>  15
<212>  PRT
<213>  Artificial

<220>
<223>  synthetic peptide

<400>  98

Arg Arg Pro Pro Gln Gly Ser Gln Thr His Gln Val Ser Leu Ser
1               5                   10                  15


<210>  99
<211>  15
<212>  PRT
<213>  Artificial

<220>
<223>  synthetic peptide

<400>  99

Gly Ser Gln Thr His Gln Val Ser Leu Ser Lys Gln Pro Thr Ser
1               5                   10                  15


<210>  100
<211>  15

<212>  PRT
<213>  Artificial

<220>
<223>  synthetic peptide

<400>  100

Gln Val Ser Leu Ser Lys Gln Pro Thr Ser Gln Ser Arg Gly Asp
1               5                   10                  15


<210>  101
<211>  15
<212>  PRT
<213>  Artificial

<220>
<223>  synthetic peptide

<400>  101

Lys Gln Pro Thr Ser Gln Ser Arg Gly Asp Pro Thr Gly Pro Lys
1               5                   10                  15


<210>  102
<211>  15
<212>  PRT
<213>  Artificial

<220>
<223>  synthetic peptide

<400>  102

Gln Ser Arg Gly Asp Pro Thr Gly Pro Lys Glu Gln Lys Lys Lys
1               5                   10                  15


<210>  103
<211>  20
<212>  PRT
<213>  Artificial

<220>
<223>  synthetic peptide

<400>  103

Gly Gly Lys Trp Ser Lys Ser Ser Val Val Gly Trp Pro Thr Val Arg
1               5                   10                  15


Glu Arg Met Arg
            20


<210>  104
<211>  20
<212>  PRT
<213>  Artificial

<220>
<223>  synthetic peptide

<400>  104

Gly Trp Pro Thr Val Arg Glu Arg Met Arg Arg Ala Glu Pro Ala Ala

1               5                   10                  15

Asp Gly Val Gly
            20

<210>  105
<211>  20
<212>  PRT
<213>  Artificial

<220>
<223>  synthetic peptide

<400>  105

Arg Ala Glu Pro Ala Ala Asp Gly Val Gly Ala Ala Ser Arg Asp Leu
1               5                   10                  15

Glu Lys His Gly
            20

<210>  106
<211>  20
<212>  PRT
<213>  Artificial

<220>
<223>  synthetic peptide

<400>  106

Ala Ala Ser Arg Asp Leu Glu Lys His Gly Ala Ile Thr Ser Ser Asn
1               5                   10                  15

Thr Ala Ala Thr
            20

<210>  107
<211>  20
<212>  PRT
<213>  Artificial

<220>
<223>  synthetic peptide

<400>  107

Ala Ile Thr Ser Ser Asn Thr Ala Ala Thr Asn Ala Ala Cys Ala Trp
1               5                   10                  15

Leu Glu Ala Gln
            20

<210>  108
<211>  20
<212>  PRT
<213>  Artificial

<220>
<223>  synthetic peptide

<400>  108

Asn Ala Ala Cys Ala Trp Leu Glu Ala Gln Glu Glu Glu Glu Val Gly
1               5                   10                  15

Phe Pro Val Thr
            20

<210>  109
<211>  20
<212>  PRT
<213>  Artificial

<220>
<223>  synthetic peptide

<400>  109

Glu Glu Glu Glu Val Gly Phe Pro Val Thr Pro Gln Val Pro Leu Arg
1               5                   10                  15

Pro Met Thr Tyr
            20

<210>  110
<211>  20
<212>  PRT
<213>  Artificial

<220>
<223>  synthetic peptide

<400>  110

Pro Gln Val Pro Leu Arg Pro Met Thr Tyr Lys Ala Ala Val Asp Leu
1               5                   10                  15

Ser His Phe Leu
            20

<210>  111
<211>  20
<212>  PRT
<213>  Artificial

<220>
<223>  synthetic peptide

<400>  111

Lys Ala Ala Val Asp Leu Ser His Phe Leu Lys Glu Lys Gly Gly Leu
1               5                   10                  15

Glu Gly Leu Ile
            20

<210>  112
<211>  20
<212>  PRT
<213>  Artificial

<220>
<223>  synthetic peptide

<400>  112

Lys Glu Lys Gly Gly Leu Glu Gly Leu Ile His Ser Gln Arg Arg Gln
1               5                   10                  15

Asp Ile Leu Asp
            20

<210>  113
<211>  20
<212>  PRT
<213>  Artificial

<220>
<223>  synthetic peptide

<400>  113

His Ser Gln Arg Arg Gln Asp Ile Leu Asp Leu Trp Ile Tyr His Thr
1               5                   10                  15

Gln Gly Tyr Phe
            20

<210>  114
<211>  20
<212>  PRT
<213>  Artificial

<220>
<223>  synthetic peptide

<400>  114

Leu Trp Ile Tyr His Thr Gln Gly Tyr Phe Pro Asp Trp Gln Asn Tyr
1               5                   10                  15

Thr Pro Gly Pro
            20

<210>  115
<211>  20
<212>  PRT
<213>  Artificial

<220>
<223>  synthetic peptide

<400>  115

Pro Asp Trp Gln Asn Tyr Thr Pro Gly Pro Gly Val Arg Tyr Pro Leu
1               5                   10                  15

Thr Phe Gly Trp
            20

<210>  116
<211>  20

<212>  PRT
<213>  Artificial

<220>
<223>  synthetic peptide

<400>  116

Gly Val Arg Tyr Pro Leu Thr Phe Gly Trp Cys Tyr Lys Leu Val Pro
1               5                   10                  15

Val Glu Pro Asp
            20

<210>  117
<211>  20
<212>  PRT
<213>  Artificial

<220>
<223>  synthetic peptide

<400>  117

Cys Tyr Lys Leu Val Pro Val Glu Pro Asp Lys Val Glu Glu Ala Asn
1               5                   10                  15

Lys Gly Glu Asn
            20

<210>  118
<211>  20
<212>  PRT
<213>  Artificial

<220>
<223>  synthetic peptide

<400>  118

Lys Val Glu Glu Ala Asn Lys Gly Glu Asn Thr Ser Leu Leu His Pro
1               5                   10                  15

Val Ser Leu His
            20

<210>  119
<211>  20
<212>  PRT
<213>  Artificial

<220>
<223>  synthetic peptide

<400>  119

Thr Ser Leu Leu His Pro Val Ser Leu His Gly Met Asp Asp Pro Glu
1               5                   10                  15

Arg Glu Val Leu
            20

<210>  120
<211>  20
<212>  PRT
<213>  Artificial

<220>
<223>  synthetic peptide

<400>  120

Gly Met Asp Asp Pro Glu Arg Glu Val Leu Glu Trp Arg Phe Asp Ser
1               5                   10                  15

Arg Leu Ala Phe
            20

<210>  121
<211>  20
<212>  PRT
<213>  Artificial

<220>
<223>  synthetic peptide

<400>  121

Glu Trp Arg Phe Asp Ser Arg Leu Ala Phe His His Val Ala Arg Glu
1               5                   10                  15

Leu His Pro Glu
            20

<210>  122
<211>  15
<212>  PRT
<213>  Artificial

<220>
<223>  synthetic peptide

<400>  122

His His Val Ala Arg Glu Leu His Pro Glu Tyr Phe Lys Asn Cys
1               5                   10                  15

<210>  123
<211>  20
<212>  PRT
<213>  Artificial

<220>
<223>  synthetic peptide

<400>  123

Glu Pro Val Asp Pro Arg Leu Glu Pro Trp Lys His Pro Gly Ser Gln
1               5                   10                  15

Pro Lys Thr Ala
            20

<210>  124
<211>  20
<212>  PRT
<213>  Artificial

<220>
<223>  synthetic peptide

<400>  124

Lys His Pro Gly Ser Gln Pro Lys Thr Ala Cys Thr Thr Cys Tyr Cys
1               5                   10                  15

Lys Lys Cys Cys
            20

<210>  125
<211>  20
<212>  PRT
<213>  Artificial

<220>
<223>  synthetic peptide

<400>  125

Cys Thr Thr Cys Tyr Cys Lys Lys Cys Cys Phe His Cys Gln Val Cys
1               5                   10                  15

Phe Thr Thr Lys
            20

<210>  126
<211>  20
<212>  PRT
<213>  Artificial

<220>
<223>  synthetic peptide

<400>  126

Phe His Cys Gln Val Cys Phe Thr Thr Lys Ala Leu Gly Ile Ser Tyr
1               5                   10                  15

Gly Arg Lys Lys
            20

<210>  127
<211>  20
<212>  PRT
<213>  Artificial

<220>
<223>  synthetic peptide

<400>  127

Ala Leu Gly Ile Ser Tyr Gly Arg Lys Lys Arg Arg Gln Arg Arg Arg
1               5                   10                  15

Pro Pro Gln Gly

            20

<210>  128
<211>  20
<212>  PRT
<213>  Artificial

<220>
<223>  synthetic peptide

<400>  128

Arg Arg Gln Arg Arg Arg Pro Pro Gln Gly Ser Gln Thr His Gln Val
1               5                   10                  15

Ser Leu Ser Lys
            20

<210>  129
<211>  19
<212>  PRT
<213>  Artificial

<220>
<223>  synthetic peptide

<400>  129

Ser Gln Thr His Gln Val Ser Leu Ser Lys Gln Pro Thr Ser Gln Pro
1               5                   10                  15

Arg Gly Asp

<210>  130
<211>  15
<212>  PRT
<213>  Artificial

<220>
<223>  synthetic peptide

<400>  130

Gln Pro Thr Ser Gln Pro Arg Gly Asp Pro Thr Gly Pro Lys Glu
1               5                   10                  15

<210>  131
<211>  8
<212>  PRT
<213>  Artificial

<220>
<223>  synthetic peptide

<400>  131

Phe Leu Lys Glu Lys Gly Gly Leu
1               5

<210>  132
<211>  206

<212>  PRT
<213>  Artificial

<220>
<223>  synthetic peptide

<400>  132

Met Gly Gly Lys Trp Ser Lys Ser Ser Val Val Gly Trp Pro Thr Val
1               5                   10                  15

Arg Glu Arg Met Arg Arg Ala Glu Pro Ala Ala Asp Gly Val Gly Ala
            20                  25                  30

Ala Ser Arg Asp Leu Glu Lys His Gly Ala Ile Thr Ser Ser Asn Thr
        35                  40                  45

Ala Ala Thr Asn Ala Ala Cys Ala Trp Leu Glu Ala Gln Glu Glu Glu
    50                  55                  60

Glu Val Gly Phe Pro Val Thr Pro Gln Val Pro Leu Arg Pro Met Thr
65                  70                  75                  80

Tyr Lys Ala Ala Val Asp Leu Ser His Phe Leu Lys Glu Lys Gly Gly
                85                  90                  95

Leu Glu Gly Leu Ile His Ser Gln Arg Arg Gln Asp Ile Leu Asp Leu
            100                 105                 110

Trp Ile Tyr His Thr Gln Gly Tyr Phe Pro Asp Trp Gln Asn Tyr Thr
        115                 120                 125

Pro Gly Pro Gly Val Arg Tyr Pro Leu Thr Phe Gly Trp Cys Tyr Lys
    130                 135                 140

Leu Val Pro Val Glu Pro Asp Lys Val Glu Glu Ala Asn Lys Gly Glu
145                 150                 155                 160

Asn Thr Ser Leu Leu His Pro Val Ser Leu His Gly Met Asp Asp Pro
                165                 170                 175

Glu Arg Glu Val Leu Glu Trp Arg Phe Asp Ser Arg Leu Ala Phe His
            180                 185                 190

His Val Ala Arg Glu Leu His Pro Glu Tyr Phe Lys Asn Cys
        195                 200                 205

<210>  133
<211>  207
<212>  PRT
<213>  Artificial

<220>
<223>  synthetic peptide

<400>  133

Met Gly Gly Lys Trp Ser Lys Ser Ser Ile Val Gly Trp Pro Ala Val
1               5                   10                  15

Arg Glu Arg Ile Arg Arg Thr Glu Pro Ala Ala Glu Gly Val Gly Ala
            20                  25                  30

Ala Ser Gln Asp Leu Asp Lys His Gly Ala Leu Thr Ser Ser Asn Thr
        35                  40                  45

Ala His Asn Asn Ala Asp Cys Ala Trp Leu Gln Ala Gln Glu Glu Glu
    50                  55                  60

Glu Glu Val Gly Phe Pro Val Arg Pro Gln Val Pro Leu Arg Pro Met
65                  70                  75                  80

Thr Tyr Lys Ala Ala Phe Asp Leu Ser Phe Phe Leu Lys Glu Lys Gly
                85                  90                  95

Gly Leu Glu Gly Leu Ile Tyr Ser Lys Lys Arg Gln Glu Ile Leu Asp
            100                 105                 110

Leu Trp Val Tyr His Thr Gln Gly Phe Phe Pro Asp Trp Gln Asn Tyr
        115                 120                 125

Thr Pro Gly Pro Gly Val Arg Tyr Pro Leu Thr Phe Gly Trp Cys Phe
    130                 135                 140

Lys Leu Val Pro Val Asp Pro Arg Glu Val Glu Glu Ala Asn Lys Gly
145                 150                 155                 160

Glu Asn Asn Cys Leu Leu His Pro Met Ser Gln His Gly Met Glu Asp
                165                 170                 175

Glu Asp Arg Glu Val Leu Lys Trp Lys Phe Asp Ser Ser Leu Ala Arg
            180                 185                 190

Arg His Met Ala Arg Glu Leu His Pro Glu Tyr Tyr Lys Asp Cys
        195                 200                 205

<210>  134
<211>  207
<212>  PRT
<213>  Artificial

<220>
<223>  synthetic peptide

<400>  134

Met Gly Gly Lys Trp Ser Lys Cys Ser Ile Val Gly Trp Pro Ala Val
1               5                   10                  15

Arg Glu Arg Met Arg Arg Thr Glu Pro Ala Ala Glu Gly Val Gly Ala
            20                  25                  30

Ala Ser Gln Asp Leu Asp Lys His Gly Ala Leu Thr Ser Ser Asn Thr
        35                  40                  45

Ala Ala Asn Asn Ala Asp Cys Ala Trp Leu Glu Ala Gln Glu Glu Glu
    50                  55                  60

Glu Glu Val Gly Phe Pro Val Arg Pro Gln Val Pro Leu Arg Pro Met
65                  70                  75                  80

Thr Tyr Lys Ala Ala Phe Asp Leu Ser Phe Phe Leu Lys Glu Lys Gly
                85                  90                  95

Gly Leu Glu Gly Leu Ile Tyr Ser Lys Lys Arg Gln Glu Ile Leu Asp
            100                 105                 110

Leu Trp Val Tyr His Thr Gln Gly Tyr Phe Pro Asp Trp Gln Asn Tyr
        115                 120                 125

Thr Pro Gly Pro Gly Val Arg Tyr Pro Leu Thr Phe Gly Trp Cys Phe
    130                 135                 140

Lys Leu Val Pro Val Asp Pro Arg Glu Val Glu Glu Ala Asn Glu Gly
145                 150                 155                 160

Glu Asn Asn Cys Leu Leu His Pro Met Ser Gln His Gly Met Glu Asp
                165                 170                 175

Glu His Arg Glu Val Leu Lys Trp Lys Phe Asp Ser His Leu Ala Arg
            180                 185                 190

Arg His Met Ala Arg Glu Leu His Pro Glu Tyr Tyr Lys Asp Cys
        195                 200                 205

<210>  135
<211>  8
<212>  PRT
<213>  Artificial

<220>
<223>  synthetic peptide

<400>  135

Trp Pro Thr Val Arg Glu Arg Met
1               5

<210>  136
<211>  9
<212>  PRT
<213>  Artificial

<220>
<223>  synthetic peptide

<400>  136

Ala Leu Thr Ser Ser Asn Thr Ala Ala
1               5


<210>  137
<211>  10
<212>  PRT
<213>  Artificial

<220>
<223>  synthetic peptide

<400>  137

Phe Pro Val Thr Pro Gln Val Pro Leu Arg
1               5                   10


<210>  138
<211>  9
<212>  PRT
<213>  Artificial

<220>
<223>  synthetic peptide

<400>  138

Phe Pro Val Arg Pro Gln Val Pro Leu
1               5


<210>  139
<211>  11
<212>  PRT
<213>  Artificial

<220>
<223>  synthetic peptide

<400>  139

Arg Pro Gln Val Pro Leu Arg Pro Met Thr Tyr
1               5                   10


<210>  140
<211>  10
<212>  PRT
<213>  Artificial

<220>
<223>  synthetic peptide

<400>  140

Gln Val Pro Leu Arg Pro Met Thr Tyr Lys
1               5                   10


<210>  141
<211>  8
<212>  PRT
<213>  Artificial

<220>
<223>  synthetic peptide

<400>  141

Val Pro Leu Arg Pro Met Thr Tyr
1               5


<210>  142
<211>  9
<212>  PRT
<213>  Artificial

<220>
<223>  synthetic peptide

<400>  142

Arg Pro Met Thr Tyr Lys Ala Ala Leu
1               5


<210>  143
<211>  9
<212>  PRT
<213>  Artificial

<220>
<223>  synthetic peptide

<400>  143

Ala Val Asp Leu Ser His Phe Leu Lys
1               5


<210>  144
<211>  8
<212>  PRT
<213>  Artificial

<220>
<223>  synthetic peptide

<400>  144

Phe Leu Lys Glu Lys Gly Gly Leu
1               5


<210>  145
<211>  10
<212>  PRT
<213>  Artificial

<220>
<223>  synthetic peptide

<400>  145

Arg Arg Gln Asp Ile Leu Asp Leu Trp Ile
1               5                   10


<210>  146
<211>  10
<212>  PRT
<213>  Artificial

<220>
<223>  synthetic peptide

<400>  146

Thr Pro Gly Pro Gly Val Arg Tyr Pro Leu
1               5                   10


<210>  147
<211>  9
<212>  PRT
<213>  Artificial

<220>
<223>  synthetic peptide

<400>  147

Tyr Pro Leu Thr Phe Gly Trp Cys Tyr
1               5


<210>  148
<211>  10
<212>  PRT
<213>  Artificial

<220>
<223>  synthetic peptide

<400>  148

Pro Leu Thr Phe Gly Trp Cys Phe Lys Leu
1               5                   10


<210>  149
<211>  9
<212>  PRT
<213>  Artificial

<220>
<223>  synthetic peptide

<400>  149

Leu Thr Phe Gly Trp Cys Phe Lys Leu
1               5


<210>  150
<211>  10
<212>  PRT
<213>  Artificial

<220>
<223>  synthetic peptide

<400>  150

Val Leu Glu Trp Arg Phe Asp Ser Arg Leu
1               5                   10